# EUROPÄISCHE PATENTSCHRIFT

(11) **EP 3 025 196 B1**
(45) Veröffentlichungstag und Bekanntmachung des Hinweises auf die Patenterteilung: **27.02.2019**
(21) Anmeldenummer: 14745108.2
(22) Anmeldetag: 25.07.2014
(51) Int. Cl.: G03F 7/00, G03F 7/20, G03F 7/40, A61L 31/02, A61L 31/08, A61F 2/82, A61B 17/00

(54) **VERFAHREN ZUR HERSTELLUNG EINER MEDIZINISCHEN VORRICHTUNG ODER EINER VORRICHTUNG MIT STRUKTURELEMENTEN, VERFAHREN ZUM MODIFIZIEREN DER OBERFLÄCHE EINER MEDIZINISCHEN VORRICHTUNG ODER EINER VORRICHTUNG MIT STRUKTURELEMENTEN, MEDIZINISCHE VORRICHTUNG UND SCHICHTVERBUND MIT EINEM SUBSTRAT**
METHOD FOR PRODUCING A MEDICAL DEVICE OR A DEVICE WITH STRUCTURE ELEMENTS, METHOD FOR MODIFYING THE SURFACE OF A MEDICAL DEVICE OR OF A DEVICE WITH STRUCTURE ELEMENTS, MEDICAL DEVICE AND LAMINATED COMPOSITE WITH A SUBSTRATE
PROCÉDÉ DE PRODUCTION D'UN DISPOSITIF MÉDICAL OU D'UN DISPOSITIF MUNI D'ÉLÉMENTS DE STRUCTURE, PROCÉDÉ PERMETTANT DE MODIFIER LA SURFACE D'UN DISPOSITIF MÉDICAL OU D'UN DISPOSITIF MUNI D'ÉLÉMENTS DE STRUCTURE, DISPOSITIF MÉDICAL ET COMPOSITE STRATIFIÉ MUNI D'UN SUBSTRAT

(30) Priorität: 25.07.2013 DE 102013107947
(43) Veröffentlichungstag der Anmeldung: 01.06.2016
(73) Patentinhaber: Acquandas GmbH, 24143 Kiel (DE)
(72) Erfinder: LIMA DE MIRANDA, Rodrigo, 24143 Kiel (DE)
(74) Vertreter: Kilchert, Jochen
(86) Internationale Anmeldenummer: PCT/EP2014/066006
(87) Internationale Veröffentlichungsnummer: WO 2015/011253

(56) Entgegenhaltungen:
- EP-A1- 2 133 306
- EP-A2- 1 304 309
- EP-A2- 2 395 395
- WO-A1-00/73241
- WO-A1-98/45506
- WO-A1-2005/098486
- WO-A1-2007/089204
- WO-A1-2008/000467
- WO-A2-03/088340
- DE-A1- 10 065 013
- DE-A1-102010 024 498

## Beschreibung

Die Erfindung betrifft ein Verfahren zur Herstellung einer medizinischen Vorrichtung mit Strukturelementen oder einer Vorrichtung mit Strukturelementen, ein Verfahren zum Modifizieren der Oberfläche einer medizinischen Vorrichtung oder einer Vorrichtung mit Strukturelementen, eine medizinische Vorrichtung mit scharfkantiger Struktur und einen Schichtverbund mit einem Substrat.

Medizinische Vorrichtungen, bspw. Stents, weisen in der Regel komplexe Strukturen auf, durch die die medizinische Vorrichtung eine besonders hohe Flexibilität erreicht. Die Flexibilität ermöglicht den Transport im Katheter und die Positionierung der Vorrichtung innerhalb eines Körperhohlorgans. Sie begünstigt ferner das Verhalten der medizinischen Vorrichtung in eng gekrümmten Gefäßen. Zur Herstellung derartiger medizinischer Vorrichtungen, insbesondere der Struktur, werden verschiedene Verfahren verwendet. Neben dem Flechten von Drahtelementen werden die Vorrichtungen auch aus Vollmaterial durch Laser-Verfahren geschnitten. Aus US 2002/0091438 A1 ist es ferner bekannt, ein Ätzverfahren zur Bildung der Strukturen zu nutzen. Hierbei werden Strukturen durch die Bildung von Freiräumen in einer dünnen Materialplatte erzeugt. Die Freiräume werden dabei durch das Ätzverfahren gebildet.

Bei dem in der US 2002/0091438 A1 beschriebenen Verfahren wird zunächst ein Rohr mit einer gleichmäßig dicken Fotolackschicht überzogen. Anschließend wird der Fotolack durch UV-Licht in den Bereichen ausgehärtet, an denen die Stege der herzustellenden medizinischen Vorrichtung, bspw. Stent, angeordnet sind. Der ausgehärtete Fotolack zeichnet also die spätere Struktur des Stents ab. Im folgenden Schritt wird das beschichtete Rohr für eine bestimmte Dauer in ein Bad mit einem Lösemittel getaucht. In dem Lösemittel lösen sich die nichtausgehärteten Stellen der Fotolackschicht, so dass in diesen Bereichen wieder das Material des Rohrs in Erscheinung tritt. In einem folgenden elektrochemischen Ätzprozess werden die freigelegten Stellen des Rohrs entfernt. Es bleiben somit nur noch die mit der ausgehärteten Fotolackschicht überzogenen Stege des Stents übrig.

Aus WO 2008/000467 A1 ist ein Verfahren zur Herstellung strukturierter Schichten aus Titan und Nickel bekannt. Dabei werden auf ein Substrat u.a. Schichten aus einem ersten und zweiten Opfermaterial sowie eine Fotolackschicht aufgetragen.
EP 2 133 306 A1 offenbart ein Verfahren zur Herstellung polymerer Mikroaktuatoren. Auf ein bereitgestelltes Substrat wird eine Fotolackschicht aufgetragen, wobei diese anschließend strukturiert wird. Es folgt das Abscheiden eines Opfermaterials, sodass in Freiräumen der Fotolackschicht ein erstes Maskenelement gebildet wird. Die Fotolackschicht wird anschließend entfernt.
WO 2007/089204 A1 betrifft ein Verfahren zur Herstellung mikroelektromechanischer Systeme, wohingegen WO 03/088340 A2 ein Verfahren zur Herstellung strukturierter Schichten auf Substraten offenbart. Dabei wird eine negativ strukturierte erste Beschichtung auf einer Substratoberfläche hergestellt und zumindest eine weitere Schicht aus einem Material mit glasartiger Struktur aufgebracht. Anschließend erfolgt eine zumindest teilweise Entfernung der ersten Schicht.
Aus EP 1 304 309 A2 ist ein Verfahren zum Herstellen eines mikromechanischen Bauelements mit einer beweglichen Struktur bekannt, wobei das Verfahren darauf beruht, dass die bewegliche Struktur teilweise mit Fotolack fixiert wird, bevor die bewegliche Struktur freigeätzt wird.

In DE 10 2010 024 498 A1 wird ein Verfahren zur Herstellung einer dreidimensionalen Struktur aus aufeinanderfolgenden Schichten offenbart. Das Erstellen jeder Schicht umfasst ein Abscheiden eines Opfermaterials mittels eines elektrochemischen Prozesses und das Abscheiden des Strukturmaterials mittels physikalischer Gasphasenabscheidung.
DE 100 65 013 A1 offenbart ein Herstellungsverfahren für ein mikromechanisches Bauelement. Es beruht auf Verfahrensschritten der Halbleitertechnik, wobei nur wenige Schichten und Fotolithografieschritte erforderlich sind. Aus WO 00/73241 A1 ist es bekannt, dass zur Herstellung keramischer Mikrostrukturen das Ausbilden von Opferschichten vorgesehen sein kann.

Der vorliegenden Erfindung liegt die Aufgabe zur Grunde, ein verbessertes Verfahren zur Herstellung einer medizinischen Vorrichtung oder einer Vorrichtung mit Strukturelementen anzugeben, bei dem die Kontur der Strukturelemente und die Materialeigenschaften besser beeinflusst werden können. Überdies liegt der Erfindung die Aufgabe zu Grunde, ein Verfahren zum Modifizieren der Oberfläche einer medizinischen Vorrichtung mit Strukturelementen oder einer Vorrichtung mit Strukturelementen anzugeben. Ferner soll mit der vorliegenden Erfindung eine medizinische Vorrichtung angeben werden, die zum Schneiden von Gewebe oder Ablagerungen in Gefäßen geeignet ist. Mit der Erfindung soll ferner ein Schichtverbund mit einem Substrat und einer Opfermaterialschicht für die Herstellung einer medizinischen Vorrichtung oder einer Vorrichtung mit Strukturelementen angegeben werden.

Erfindungsgemäß wird diese Aufgabe mit Blick auf das Verfahren zur Herstellung einer medizinischen Vorrichtung durch das Verfahren nach Anspruch 1, mit Blick auf das Verfahren zur Herstellung einer Vorrichtung mit Strukturelementen durch das Verfahren nach Anspruch 2 und mit Blick auf das Verfahren zum Modifizieren der Oberfläche einer medizinischen Vorrichtung durch das Verfahren nach Anspruch 14 und mit Blick auf das Verfahren zum Modifizieren der Oberfläche einer Vorrichtung mit Strukturelementen durch das Verfahren nach Anspruch 15 gelöst. Mit Blick auf die medizinische Vorrichtung wird die Aufgabe durch den Anspruch 16 gelöst. Mit Blick auf den Schichtverbund wird die Aufgabe durch den Gegenstand nach Anspruch 17 oder 18 gelöst.

Die Erfindung beruht auf dem Gedanken, ein Verfahren zur Herstellung einer medizinischen Vorrichtung mit Strukturelementen anzugeben, die folgende Verfahrensschritte umfasst:
- Bereitstellen eines Substrates;
- Abscheiden einer Schicht aus einem Opfermaterial auf dem Substrat;
- Auftragen einer Fotolackschicht auf die Opfermaterialschicht,
- Strukturieren der Fotolackschicht gemäß der Form der herzustellenden Strukturelemente, so dass erste Freiräume gebildet werden, die auf der Substrat abgewandten Seite offen sind und durch Seitenflächen der Fotolackschicht begrenzt werden, wobei ein Winkel, der 45° bis kleiner als 90° beträgt, zwischen den Seitenflächen und dem Substrat eingestellt wird;
- Abscheiden von Opfermaterial, so dass in den ersten Freiräumen erste Maskenelemente aus Opfermaterial gebildet werden, die an die Innenkontur der ersten Freiräume angepasst sind;
- Entfernen der Fotolackschicht, so dass zweite Freiräume zwischen den ersten Maskenelementen gebildet werden;
- Abscheiden, insbesondere physikalisches Gasphasenabscheiden, eines ersten Materials der herzustellenden Vorrichtung in den zweiten Freiräumen und auf der Oberseite der ersten Maskenelemente; und
- Abtragen des Opfermaterials und/oder Durchführen weiterer Prozessschritte.

Die Erfindung beruht außerdem auf dem Gedanken, ein Verfahren zur Herstellung einer Vorrichtung mit Strukturelementen, insbesondere zur Herstellung von Aktoren und/oder Mikropumpen, linearen Aktoren im Bereich der Mikrosystemtechnik (MEMS) und/oder Aktoren für minimal invasive Instrumente und/oder Mikroaktoren und/oder flexible Mikroantennen, anzugeben, wobei das Verfahren die bereits genannten Verfahrensschritte umfasst.

Die herzustellenden Mikropumpen können beispielsweise in der Fluidik verwendet bzw. eingesetzt werden. Die herzustellenden Aktoren für minimal invasive Instrumente können beispielsweise im Zusammenhang mit lenkbaren Hypotubes und/oder Kathetern verwendet bzw. eingesetzt werden.

Die folgende Beschreibung stellt auf einzelne Verfahrensschritte der erfindungsgemäßen Verfahren ab, d.h. diese betreffen sowohl das Verfahren zur Herstellung einer medizinischen Vorrichtung, als auch das Verfahren zur Herstellung einer Vorrichtung mit Strukturelementen. Somit können die einzeln beschriebenen Verfahrensschritte auch bei der Herstellung einer Vorrichtung mit Strukturelementen erfolgen, obwohl möglicherweise explizit auf eine medizinische Vorrichtung verwiesen wird.

Das bzw. die Verfahren hat bzw. haben den Vorteil, dass beim Abscheiden, insbesondere beim physikalischen Gasphasenabscheiden die Materialeigenschaften gezielt eingestellt werden können. So ist es bspw. möglich, die Gefügeeigenschaften in einem größeren Bereich einzustellen, als dies schmelzmetallurgisch möglich ist. Verunreinigungen des Materials können besser vermieden werden, da das Abscheiden üblicherweise im Vakuum erfolgt. Überdies entfallen zusätzliche Wärmebehandlungsschritte, die im Stand der Technik erforderlich sind, um die beim Walzen des Vollmaterials, bzw. allgemein bei der mechanischen Behandlung des Vollmaterials entstehenden Gefügeänderungen zu beeinflussen.

Zudem ermöglicht die Erfindung unterschiedliche Profilierungen der Strukturelemente durch die Ausbildung der Maskenelemente, so dass sowohl die Geometrie als auch die Materialeigenschaften der Strukturelemente erfindungsgemäß eingestellt werden können. Die Kombination von PVD-Verfahren mit den erfindungsgemäß strukturierten Maskenelementen ermöglicht die Herstellung freitragender profilierter Strukturelemente, bspw. Stege eines Stents, die zusätzlich zur Geometrie hervorragende Materialeigenschaften aufweisen.

Grundsätzlich kann das Substrat aus jedem beliebigen Material gebildet sein. Das Substrat sollte eine möglichst glatte Oberfläche aufweisen, da die Eigenschaften der Oberfläche des Substrats die Oberfläche der herzustellenden medizinischen Vorrichtung beeinflussen. Erstrebenswert ist eine möglichst glatte Oberfläche, so dass das Substrat bevorzugt Materialien aufweist, die diese Eigenschaften mit sich bringen. Vorzugsweise kann das Substrat aus Glas, Silizium, Quarz bestehen. Das erfindungsgemäße Verfahren zur Herstellung der medizinischen Vorrichtung umfasst das optionale Abscheiden einer ersten Schicht aus einem Opfermaterial auf dem Substrat. Optional ist dieses Merkmal deshalb, da bereits das bereitgestellte Substrat aus dem Opfermaterial hergestellt sein kann. In diesem Fall ist das Abscheiden der ersten Opfermaterialschicht nicht zwingend erforderlich.

Vorzugsweise ist die Opfermaterialschicht elektrisch leitend. Sie kann bspw. aus Kupfer gebildet sein. Die Dicke der Schicht beträgt > 1 nm.

Im folgenden Schritt wird eine Fotolackschicht auf das Substrat bzw. auf die optionale Opfermaterialschicht aufgetragen. Der verwendete Lack kann ein Fotolack sein, der im Rahmen eines Lithografieverfahrens strukturiert werden kann. Vorzugsweise wird ein strahlungsempfindlicher Lack verwendet, der beispielsweise durch Belichtung mit UV-Licht aushärtet. Anschließend wird die Fotolackschicht strukturiert, so dass die Fotolackschicht bzw. die Struktur der strukturierten Fotolackschicht die Form der herzustellenden Strukturelemente der medizinischen Vorrichtung nachbildet. Die Strukturierung bzw. die Struktur des Fotolacks entsteht durch unterschiedliche Belichtung und damit Aushärten des Fotolacks.

Die Struktur wird dadurch erzeugt, dass der Fotolack an den Stellen und Bereichen ausgehärtet wird, an denen später im Verfahren das Material der herzustellenden medizinischen Vorrichtung abgeschieden wird. Das Aushärten kann durch verschiedene Belichtungsverfahren durchgeführt werden. Gängige Belichtungsverfahren sind beispielsweise die Elektronenstrahl-Lithographie sowie die Röntgenstrahl-Lithographie. Bevorzugt wird die optische Lithographie (Fotolithographie). Während der Belichtung des Fotolacks wird eine Fotomaske verwendet, die verschiedene Bereiche des Fotolacks abdeckt und damit Schatten bildet. Die Schattenbildung führt dazu, dass das UV-Licht nicht auf den Fotolack auftrifft und somit auch nicht zur Aushärtung des Fotolacks führt. Somit trägt die Form der Fotomaske dazu bei, die vorbestimmte Struktur der medizinischen Vorrichtung zu beeinflussen. Damit entspricht die Struktur der Fotomaske der Struktur der herzustellenden medizinischen Vorrichtung. Ferner können Parameter des Belichtungsverfahrens, beispielsweise Belichtungszeit, Intensität der Belichtung oder Abstand der Belichtungsquelle zur medizinischen Vorrichtung, eingestellt werden. Als Belichtungsmittel wird vorzugsweise ultraviolettes Licht (UV-Licht) verwendet. Der Einfallswinkel des UV-Lichts kann ebenfalls eingestellt werden, so dass eine vom Einfallswinkel des UV-Lichts abhängige Aushärtung des Fotolacks erfolgt. Dadurch ist der Winkel zwischen den Seitenflächen der Fotolackschicht und dem Substrat bzw. dem ersten Opfermaterial einstellbar.

Nach der Aushärtung des Fotolacks werden die nicht ausgehärteten Bereiche des Fotolacks entfernt. Auf dem Substrat verbleibt eine gehärtete Fotolackschicht, die zumindest teilweise den Strukturelementen der medizinischen Vorrichtung entspricht. Die Seitenflächen der Fotolackschicht entsprechen ebenfalls zumindest teilweise den Seitenflächen der Strukturelemente der medizinischen Vorrichtung. Die entfernten Bereiche der Fotolackschicht bilden erste Freiräume.

Im folgenden Schritt wird ein weiteres Opfermaterial abgeschieden. Das weitere Opfermaterial kann aus dem gleichen Material bestehen, aus dem auch die erste Opfermaterialschicht besteht. Vorzugsweise umfasst das zweite Opfermaterial ein elektrisch leitendes Material, insbesondere Kupfer. Die Abscheidung des Opfermaterials erfolgt im Wesentlichen in den ersten Freiräumen. Dabei entstehen aus dem Opfermaterial erste Maskenelemente. Diese Maskenelemente sind an die Innenkontur der ersten Freiräume angepasst. Die Gestalt der Maskenelemente beeinflusst die Profilform der Strukturelemente der herzustellenden Vorrichtung. Das Abscheiden des Opfermaterials kann im Prinzip durch jedes beliebige Abscheideverfahren erfolgen. Vorzugsweise wird jedoch ein elektrochemisches Abscheideverfahren verwendet, insbesondere ein galvanisches Abscheideverfahren. Das galvanische Abscheidungsverfahren ermöglicht das Wachstum der Maskenelemente derart, dass die Maskenelemente an die Innenkontur der ersten Freiräume angepasst sind.

In einem folgenden Schritt wird die Fotolackschicht entfernt. Dadurch entstehen zweite Freiräume zwischen den ersten Maskenelementen.

Im nächsten Verfahrensschritt wird ein erstes Material der herzustellenden Vorrichtung abgeschieden. Die Abscheidung erfolgt vorzugsweise durch ein physikalisches Gasphasen-Abscheideverfahren, wie Sputtern. Sputtern ist besonders gut geeignet, um die Materialeigenschaften der Vorrichtung, insbesondere der Strukturelemente, gezielt einzustellen.

Dabei wird das erste Material der herzustellenden Vorrichtung in die zweiten Freiräume sowie auf die Oberseite der ersten Maskenelemente abgeschieden. Mit anderen Worten werden die zweiten Freiräume mit dem ersten Material der herzustellenden Vorrichtung aufgefüllt. Das Abscheiden des ersten Materials auf der Oberseite der ersten Maskenelemente erzeugt ein nahezu rechteckiges Profil der abgeschiedenen Schicht. Dagegen wird in den zweiten Freiräumen das erste Material derart abgeschieden, dass sich profilierte Elemente bilden. Die Profilierung der Strukturelemente wird durch die Neigung der Wände der ersten Maskenelemente beeinflusst.

Im nächsten Verfahrensschritt wird das Opfermaterial abgetragen. Alternativ oder auch zusätzlich können weitere Prozessschritte durchgeführt werden. Die Abtragung des Opfermaterials kann durch verschiedene Ätzverfahren vorgenommen werden. Bekannt sind beispielsweise nasschemisches Ätzen sowie Trockenätzverfahren. Durch die Ätzverfahren werden zwar die Opfermaterialien abgetragen, jedoch wird das erste Material der herzustellenden Vorrichtung nicht angegriffen. Nach dem Abtragen des Opfermaterials bleibt daher nur das erste Material der herzustellenden Vorrichtung bestehen.
Erfindungsgemäß ist vorgesehen, dass der Winkel zwischen einer Seitenfläche und dem Substrat von 45 Grad bis kleiner als 90 Grad beträgt. Ferner können die beiden Seitenflächen eines Freiraumes unterschiedliche Winkel zum Substrat aufweisen. Diese Ausgestaltung führt dazu, dass die herzustellende medizinische Vorrichtung Strukturelemente aufweist, die im Querschnitt asymmetrisch sind. Eine erste Seitenfläche des Strukturelements kann stärker geneigt sein als die zweite Seitenfläche des Strukturelements.

Gemäß der Ausgestattung der Erfindung ergibt sich dass die Seitenflächen jeweils eines Freiraums trichterförmig geöffnet sind. Der Freiraum, der durch die Strukturierung des Fotolacks entsteht, verjüngt sich zum Substrat hin und der Abstand der Seitenflächen nimmt ab. Der weitere Freiraum, der durch die Maskenelemente begrenzt ist, ist zumindest abschnittsweise das Negativ des Freiraums im Fotolack und verbreitert sich zum Substrat hin.
Gemäß einer bevorzugten Ausgestaltung der Erfindung ist vorgesehen, dass die ersten Maskenelemente Flanken aufweisen, die entsprechend dem Winkel der Seitenflächen geneigt sind.
Durch die Abscheidung des Opfermaterials werden die Maskenelemente in den ersten Freiräumen gebildet. Mit anderen Worten wirken die Seitenflächen der Fotolackschicht wie eine formgebende Einrichtung, die die Opferschicht bei der Abscheidung in eine bestimmte Form führt. Die Seitenflächen des Fotolacks prägen somit die Flanken des ersten Maskenelements.
Gemäß einer Ausgestaltung der vorliegenden Erfindung ist vorgesehen, dass die Fotolackschicht derart strukturiert wird, dass der Verlauf stegartiger Strukturelemente, insbesondere der Verlauf der Stege einer Gitterstruktur, abgebildet wird. Durch die Strukturierung der Fotolackschicht werden in der Fotolackschicht Gruben gebildet. In diese Gruben kann dann das zweite Opfermaterial abgeschieden werden. Durch die Strukturierung des Fotolacks wird daher der Verlauf der stegartigen Strukturelemente in besonders einfacher Weise bestimmt und gebildet.

Gemäß einer Ausgestaltung der vorliegenden Erfindung sind des Weiteren folgende Verfahrensschritte vorgesehen:
- Optionales Abscheiden einer weiteren Opfermaterialschicht auf das Material der herzustellenden Vorrichtung;
- Auftragen einer weiteren Fotolackschicht;
- Strukturieren der weiteren Fotolackschicht gemäß der Form einer Oberflächenprofilierung der herzustellenden Strukturelemente;
- Abscheiden von Opfermaterial, so dass wenigstens ein weiteres zweites Maskenelement gebildet wird, das an die Außenkontur der strukturierten weiteren Fotolackschicht angepasst ist;
- Entfernen der Fotolackschicht und der optionalen weiteren Opfermaterialschicht, so dass ein dritter Freiraum gebildet wird, der das Material der herzustellenden Vorrichtung freilegt und von dem weiteren Maskenelement oder von mehreren weiteren Maskenelementen begrenzt wird;
- Abscheiden, insbesondere physikalisches Gasphasenabscheiden, des ersten oder eines zweiten Materials der herzustellenden Vorrichtung im dritten Freiraum; und
   Abtragen des Opfermaterials.

Durch die Erweiterung des bisher beschriebenen Verfahrens durch die oben genannten Verfahrensschritte wird eine Profilierung der Oberfläche der medizinischen Vorrichtung und/oder der Vorrichtung mit Strukturelementen ermöglicht.

Diese Oberflächenprofilierung ist besonders im Zusammenhang mit dem vorstehend beschriebenen Verfahren vorteilhaft, da diese in einem Arbeitsgang direkt im Anschluss an die Herstellung der Strukturelemente durchgeführt werden kann.

Zunächst wird optional eine weitere Opfermaterialschicht auf das Material der herzustellenden Vorrichtung abgeschieden. Anschließend wird eine weitere Fotolackschicht aufgetragen und diese strukturiert. Hierbei entspricht die Strukturierung der weiteren Fotolackschicht dem Oberflächenprofil der herzustellenden Strukturelemente. Die Strukturierung der Fotolackschicht erfolgt dabei im Wesentlichen wie die Strukturierung der ersten Fotolackschicht, die bereits oben beschrieben wurde.

Anschließend wird ein weiteres Opfermaterial abgeschieden, so dass ein Maskenelement gebildet wird. Durch die Außenkonturen der strukturierten Fotolackschicht wird dem Maskenelement eine Form aufgeprägt, die komplementär zur strukturierten Fotolackschicht gebildet ist.

Im nächsten Schritt werden die Fotolackschicht und die optionale weitere Opfermaterialschicht entfernt, so dass ein dritter Freiraum gebildet wird. Der dritte Freiraum reicht bis zum Material der herzustellenden Vorrichtung. Somit ist der dritte Freiraum in Richtung des Substrats durch das erste Material der herzustellenden Vorrichtung begrenzt. In horizontaler Richtung, d.h. in einer Richtung parallel zum Substrat, wird der dritte Freiraum durch das weitere Maskenelement bzw. durch die mehreren weiteren Maskenelemente begrenzt.

In einem nächsten Schritt wird das erste oder auch ein zweites Material der herzustellenden Vorrichtung in den dritten Freiraum abgeschieden. Dabei erfolgt die Abscheidung vorzugsweise durch ein physikalisches Gasphasen-Abscheideverfahren, insbesondere Sputtern.

Anschließend wird das verbleibende Opfermaterial abgetragen bzw. entfernt.

Es ist auch möglich, die Oberflächenprofilierung im Zusammenhang mit einem anderen Herstellungsverfahren, bspw. mit einem herkömmlichen Ätzverfahren oder Laserschneidverfahren anzuwenden. In diesem Fall wird die anderweitig hergestellte, fertige Vorrichtung der Oberflächenprofilierung unterzogen, die in den nebengeordneten Ansprüchen 14 und/oder 15 beansprucht werden, wobei nur die Terminologie angepasst ist. Beispielsweise kann auf diese Weise auf einen Stent eine Struktur aufgesputtert werden.

Gemäß einer bevorzugten Ausgestaltung der Erfindung ist vorgesehen, dass während des Strukturierens der weiteren Fotolackschicht ein Winkel zwischen den Seitenflächen der weiteren Fotolackschicht und der weiteren Opfermaterialschicht eingestellt wird. Durch diese Ausgestaltung können die Seitenflächen der Oberflächenprofilierung der herzustellenden Strukturelemente beeinflusst werden.

Dabei entspricht der Winkel der weiteren Fotolackschicht in etwa dem Winkel der herzustellenden Oberflächenprofilierung.

In einer bevorzugten Ausgestaltung der Erfindung ist vorgesehen, dass die Opfermaterialschicht, insbesondere die Opfermaterialschichten, elektrisch leitend ist, und die Maskenelemente elektrochemisch auf den elektrisch leitenden Opfermaterialschichten abgeschieden werden. Durch Verwendung eines elektrochemischen Abscheideverfahrens wird die Gestaltung der Maskenelemente vereinfacht.

Gemäß einer bevorzugten Ausgestaltung der Erfindung ist vorgesehen, dass das Material der herzustellenden Vorrichtung durch Sputtern abgeschieden wird.

Gemäß einer bevorzugten Ausgestaltung der Erfindung ist vorgesehen, dass die Höhe der Maskenelemente, insbesondere der ersten und zweiten Maskenelemente zur Beeinflussung der maximalen Höhe der herzustellenden Strukturelemente eingestellt wird. Dabei kann die maximale Höhe der Strukturelemente höher sein als die Höhe der Maskenelemente, bspw. um 1-20%. Es ist auch möglich, den Freiraum zwischen den Maskenelementen nicht komplett zu füllen, so dass die Höhe der Strukturelemente geringer als die Höhe der Maskenelemente ist bspw. um 10-20%. Ferner können die Strukturelemente bündig mit den Maskenelementen abschließen. Die Höhe der Maskenelemente bestimmt somit den Bereich, in dem sich die maximale Höhe der Strukturelemente bewegt.

Des Weiteren beeinflusst die Höhe der Maskenelemente die Form der Strukturelemente. Da die einzelnen Maskenelemente in einer Richtung vom Substrat weg breiter werden, nimmt der Abstand der oberen Seite der Maskenelemente mit zunehmender Höhe ab. Je höher die Maskenelemente sind, um so spitzer wir der Querschnitt der Strukturelemente. Der Freiraum zwischen den Maskenelementen und somit das Querschnittsprofil der Strukutrelemente nähert sich daher mit zunehmender Höhe einer Dreiecksform an.

Gemäß einer besonders bevorzugten Ausgestaltung der Erfindung ist vorgesehen, dass der Abstand der ersten Maskenelemente zur Beeinflussung der Breite der herzustellenden Strukturelemente eingestellt wird. Durch den Abstand der Maskenelemente zueinander kann die Breite der herzustellenden Strukturelemente gezielt eingestellt werden. Je größer der Abstand der Maskenelemente zueinander ist, um so breiter werden die herzustellenden Strukturelemente der medizinischen Vorrichtung. Durch den Abstand Maskenelemente zueinander wird auch die Form des Querschnitts der Strukturelemente beeinflusst. Dadurch ist somit auch die Form des Querschnitts einstellbar. Die obere Seite des Strukturelements kann durch den Abstand der Maskenelemente zueinander breiter oder schmaler gestaltet werden. Mit zunehmendem Abstand der Maskenelemente zueinander wird die obere Seite des Strukturelements breiter. Die Form des Querschnitts des Strukturelements tendiert daher zu einem Trapez. Mit geringerem Abstand der Maskenelemente zueinander wird die obere Seite des Strukturelements schmaler. Die Form des Querschnitts des Strukturelements tendiert daher zu einem Dreieck. Der Querschnitt des herzustellenden Strukturelements kann also durch die Gestalt der Maskenelemente gezielt eingestellt werden. Dabei spielen insbesondere folgende Faktoren eine Rolle: Höhe der Maskenelemente, Abstand der Maskenelemente zueinander und der Winkel der Flanken der Maskenelemente, der die Trichterform der Maskenelemente bestimmt. Durch die gezielte Einstellung dieser Faktoren wir die Querschnittsform der Strukturelemente eingestellt.

Gemäß einer bevorzugten Ausgestaltung der Erfindung ist vorgesehen, dass die ersten und/oder zweiten Maskenelemente eine Höhe von 2-100 µm aufweisen. Dadurch können die Strukturelemente der herzustellenden Vorrichtung mit einer Höhe von 2-100 µm gebildet werden.

Gemäß einer bevorzugten Ausgestaltung der Erfindung ist vorgesehen, dass das erste Material der medizinischen Vorrichtung oder der Vorrichtung mit einem Strukturelement und/oder das zweite Material der medizinischen Vorrichtung oder der Vorrichtung mit einem Strukturelement ein Formgedächtnismaterial, insbesondere Nitinol (NiTi), und/oder ein Schwermetall, insbesondere Tantal, und/oder ein elektrisch leitendes Material umfassen.

Gemäß einer bevorzugten Ausgestaltung der Erfindung ist vorgesehen, dass das erste Material der medizinischen Vorrichtung ein Formgedächtnismaterial aufweist und das zweite Material der medizinischen Vorrichtung ein Schwermetall aufweist. Dadurch kann beispielsweise eine medizinische Vorrichtung in Form eines Stents gebildet werden, die mittels des Schwermetalls beispielsweise durch Tantal, röntgendichtes Material umfasst, so dass der Stent auch innerhalb des Körpers leicht zu erfassen ist.

Das zweite Material der medizinischen Vorrichtung kann auch vollständig auf dem ersten Material der medizinischen Vorrichtung abgeschieden werden. Das zweite Material der medizinischen Vorrichtung weist keine Profilierung auf, sondern bildet eine zweite Schicht, die auf dem ersten Material der medizinischen Vorrichtung aufliegt und diese vollständig überdeckt. Anschließend kann ein drittes Material der medizinischen Vorrichtung abgeschieden werden, so dass das dritte Material wiederum das zweite Material der medizinischen Vorrichtung zumindest teilweise überdeckt. Beispielsweise kann das zweite Material der medizinischen Vorrichtung ein elektrisch leitendes Material sein, das stellenweise von dem dritten Material überdeckt ist, so dass Kontaktstellen gebildet werden, die in den freiliegenden Bereichen, welche nicht durch das dritte Material verdeckt werden, offenliegen. Die offenliegenden Stellen können somit in Kontakt mit beispielweise einer Gefäßwand stehen.

Gemäß einer nebengeordneten Ausgestaltung der Erfindung ist eine medizinische Vorrichtung vorgesehen, die durch eines der vorhergehend beschriebenen Verfahren hergestellt ist. Dabei weisen die Strukturelemente der medizinischen Vorrichtung wenigstens eine geneigte Seitenwand mit einer Schneidkante auf, die zwischen der Seitenwand und einer Grundfläche des Strukturelements gebildet ist, wobei die Schneidkante über die Fläche der Seitenwand vorsteht und eine gekrümmte Flanke aufweist, die kontinuierlich in die Fläche der Seitenwand übergeht. Die Grundfläche ist die im implantierten Zustand der Gefäßwand zugewandte Fläche (Außenfläche). Der Schneidkante wird dadurch gebildet, dass die gekrümmte Flanke mit der Grundfläche zusammenläuft und am freien Ende eine Spitze bildet. Die Spitze erstreckt sich in Längsrichtung entlang der Seitenwand. Der Krümmungsmittelpunkt der Flanke liegt außerhalb des Strukturelements (konkave Krümmung).

Die Schneidkante kann dazu verwendet werden, Ablagerungen in einem Gefäß zu entfernen. Des Weiteren kann die Schneidkante einen Widerhaken bilden, der sich in das Gewebe einschneidet, um die Vorrichtung in einer Gefäßwand zu verankern und eine Dislokation zu verhindern.

Gemäß einer nebengeordneten Ausgestaltung der Erfindung ist ein Schichtverbund mit einem Substrat und einer Opfermaterialschicht für die Herstellung einer medizinischen Vorrichtung mit Strukturelementen vorgesehen, wobei die Opfermaterialschicht auf dem Substrat angeordnet ist und auf der Opfermaterialschicht Maskenelemente aus einem Opfermaterial gebildet sind, deren Flanken einen Winkel aufweisen, der komplementär zum Winkel der Seitenwände der herzustellenden Vorrichtung gebildet ist, wobei der Abstand zwischen den Maskenelementen der Breite eines Strukturelements der herzustellenden Vorrichtung entspricht.

Gemäß einem weiteren nebengeordneten Aspekt der Erfindung ist ein Schichtverbund mit einem Substrat und einer Opfermaterialschicht für die Herstellung einer Vorrichtung mit Strukturelementen, insbesondere für die Herstellung von Aktoren und/oder Mikropumpen und/oder linearen Aktoren im Bereich der Mikrosystemtechnik und/oder Aktoren für minimal invasive Instrumente und/oder Mikroaktoren undoder flexiblen Mikroantennen, vorgesehen, wobei die Opfermaterialschicht auf dem Substrat angeordnet ist und auf der Opfermaterialschicht Maskenelemente aus einem Opfermaterial gebildet sind, deren Flanken einen Winkel aufweisen, der komplementär zum Winkel der Seitenwände der herzustellenden Vorrichtung gebildet ist, wobei der Abstand zwischen den Maskenelementen der Breite eines Strukturelements der herzustellenden Vorrichtung entspricht.

Der beschriebene Schichtverbund ermöglicht in einfacher Weise die Herstellung einer medizinischen Vorrichtung mit profilierten Strukturelementen. Die Strukturelemente weisen an den Seitenflächen Winkel auf, die den Winkeln der Flanken der Maskenelemente entsprechen.

Die Erfindung wird im Folgenden anhand von Ausführungsbeispielen unter Bezugnahme auf die beigefügten Zeichnungen näher erläutert.

Darin zeigen:
- Fig. 1: ein Substrat mit einem darauf abgeschiedenen ersten Opfermaterial;
- Fig. 2: einen Schnitt durch das Substrat mit dem abgeschiedenen Opfermaterial aus Fig. 1 mit einer strukturierten Fotolackschicht;
- Fig. 3: den Schichtverbund aus Fig. 2 mit einem abgeschiedenen zweiten Opfermaterial;
- Fig. 4: den Schichtverbund aus Fig. 3 mit der entfernten Fotolackschicht;
- Fig. 5: den Schichtverbund mit einem abgeschiedenen ersten Material der herzustellenden Vorrichtung;
- Fig. 6: die Strukturelemente der herzustellenden medizinischen Vorrichtung nach dem Abtragen des Opfermaterials;
- Fig. 7: die freistehende medizinische Vorrichtung in ihrem Endstadium;
- Fig. 8: den Schichtverbund aus Fig. 5, wobei eine weitere Opfermaterialschicht abgeschieden wurde;
- Fig. 9: den Schichtverbund aus Fig. 8, wobei strukturierter Fotolack aufgetragen wurde;
- Fig. 10: den Schichtverbund aus Fig. 9, wobei eine weitere Opfermaterialschicht aufgetragen wurde;
- Fig. 11: den Schichtverbund aus Fig. 10, wobei die Fotolackschicht entfernt wurde;
- Fig. 12: den Schichtverbund aus Fig. 11, wobei die Opfermaterialschicht abgetragen wurde;
- Fig. 13: den Schichtverbund aus Fig. 12, wobei ein zweites Material der herzustellenden Vorrichtung abgeschieden wurde;
- Fig. 14: den Schichtverbund aus Fig. 13, wobei die Opfermaterialschichten abgetragen wurden;
- Fig. 15: die hergestellte medizinische Vorrichtung mit einem Oberflächenprofil im Schnitt;
- Fig. 16: ein Lichtbild der medizinischen Vorrichtung mit einer Oberflächenprofilierung;
- Fig. 17: ein Lichtbild der Oberflächenprofilierung der hergestellten Strukturelemente in Vergrößerung;
- Fig. 18: einen Querschnitt durch ein erfindungsgemäß hergestelltes Strukturelement mit trapezförmigen Querschnitt;
- Fig. 19: einen Querschnitt durch ein erfindungsgemäß hergestelltes Strukturelement mit dreieckigem Querschnitt;
- Fig. 20: einen Querschnitt durch ein erfindungsgemäß hergestelltes Strukturelement mit trapezförmigem Querschnitt mit stärker geneigten Seitenwänden;
- Fig. 21: den Schichtverbund aus Fig. 8, wobei strukturierter Fotolack aufgetragen wurde;
- Fig. 22: den Schichtverbund aus Fig. 21, wobei eine weitere Opfermaterialschicht aufgetragen wurde;
- Fig. 23: den Schichtverbund aus Fig. 22, wobei die Fotolackschicht entfernt wurde;
- Fig. 24: den Schichtverbund aus Fig. 23, wobei die Opfermaterialschicht abschnittsweise abgetragen wurde;
- Fig. 25: den Schichtverbund aus Fig. 24, wobei ein zweites Material der herzustellenden Vorrichtung abgeschieden wurde;
- Fig. 26: den Schichtverbund aus Fig. 25, wobei die Opfermaterialschichten abgetragen wurden; und
- Fig. 27: die hergestellte (medizinische) Vorrichtung mit einem Oberflächenprofil im Schnitt.

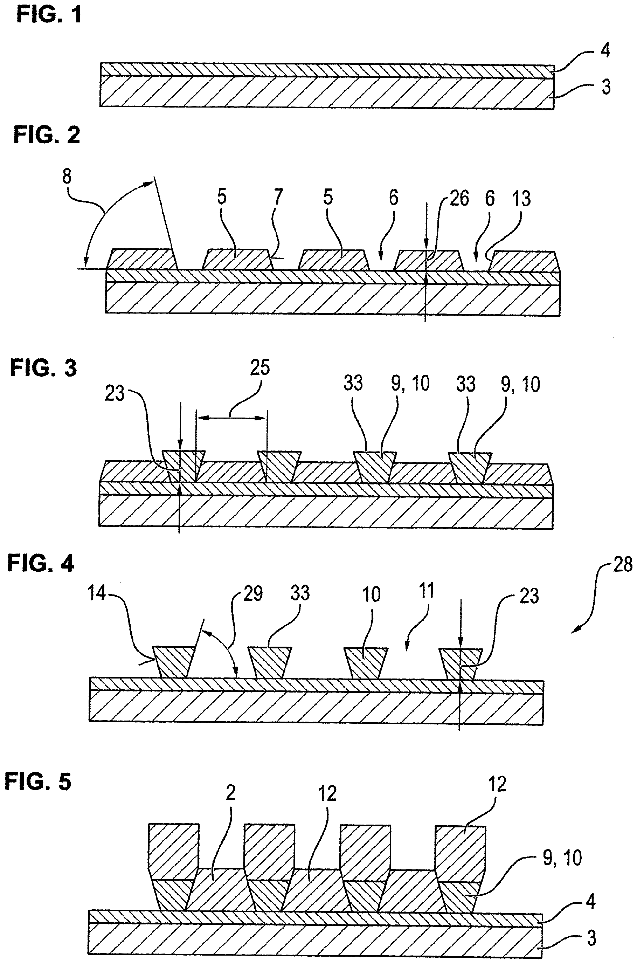

Fig. 1 zeigt das Substrat 3 mit einem abgeschiedenen Opfermaterial 4, beispielsweise Kupfer. Das Substrat weist eine glatte Oberfläche, d.h. eine Oberfläche mit einer sehr geringen Oberflächenrauigkeit, auf.

Fig. 2 zeigt den Schichtverbund aus Fig. 1, wobei dieser eine strukturierte Fotolackschicht 5 aufweist. Die Fotolackschicht 5 wird auf die Opfermaterialschicht 4 aufgetragen und strukturiert. Die strukturierte Fotolackschicht 5 weist erste Freiräume 6 auf, die durch Seitenflächen 7 begrenzt sind. Die Seitenflächen 7 bilden einen Winkel 8 zum Substrat 3. Dadurch ergibt sich eine Innenkontur 13 der Freiräume 6. Die Freiräume 6 sind auf der vom Substrat 3 abgewandten Seite offen. Die Freiräume bilden somit offene Gräben. Die Strukturierung der Fotolackschicht 5 erfolgt durch ein Belichtungsverfahren, insbesondere durch UV-Lithographie. Hierfür wird die Fotolackschicht 5 bereichsweise mit UV-Licht belichtet. Die Abgrenzungen zwischen den belichteten und nicht belichteten Bereichen des Fotolacks 5 werden durch eine nicht dargestellte Fotomaske erzeugt. Die Fotomaske schattiert bereichsweise den Fotolack, so dass das UV-Licht nicht mehr bis auf den Fotolack durchdringt. Im vorliegenden Ausführungsbeispiel bilden die Seitenflächen 7 der im Fotolack 5 ausgebildeten ersten Freiräume 6 einen Winkel 8 von etwa 60 Grad. Dieser Winkel 8 wird erzeugt, indem das UV-Licht in einem entsprechenden Winkel auf den Fotolack auftrifft.

In Fig. 3 ist ein weiterer Verfahrensschritt dargestellt, bei dem durch ein galvanisches Abscheideverfahren ein weiteres Opfermaterial auf den dargestellten Schichtverbund aus Fig. 2 abgeschieden wird. Aus Fig. 3 ist ersichtlich, dass durch das galvanische Abscheideverfahren Maskenelemente 10 mit einer Oberseite 33 gebildet werden. Die Oberseite 33 bildet eine freie Fläche, die vom Substrat abgewandt ist. Die Maskenelemente 10 füllen die Freiräume 6 auf und wachsen im vorliegenden Ausführungsbeispiel über die Höhe 26 der Fotolackschicht 5 hinaus. Die Seitenflächen 7 der Fotolackschicht 5 erzeugen während der Abscheidung des Opfermaterials 9 die geneigten Flanken 14 der ersten Maskenelemente 10. Die Maskenelemente 10 wachsen in einer Richtung senkrecht weg vom Substrat. Das jeweilige Maskenelement 10 ist an seinem dem Substrat 3 zugewandten Ende so breit, wie der Freiraum 6 auf seinem Grund bzw. an seiner zum Opfermaterial 4 angrenzenden Fläche. Das erste Maskenelement 10 wird im Verlauf der Höhe breiter und bildet einen keilförmigen Querschnitt.

Fig. 4 zeigt den Schichtverbund gemäß Fig. 3 in einem Verfahrensstadium, in dem der Fotolack entfernt wurde. Der Fotolack wird vorzugsweise mit Aceton oder einem ähnlichen Lösungsmittel entfernt. Es verbleibt das Substrat 3, das auf dem Substrat 3 aufliegende Opfermaterial 4 sowie die aus dem Opfermaterial 9 gebildeten Maskenelemente 10. Die Flanken der Maskenelemente 10 bilden zum Substrat einen Winkel 29. Der Maskenwinkel 29 ist komplementär zum Fotolackwinkel 8. Zwischen den Maskenelementen 10 sind nunmehr zweite Freiräume 11 gebildet.

Fig. 5 zeigt den Schichtverbund 28 aus Fig. 4, wobei in einem weiteren Verfahrensschritt ein erstes Material 12 der herzustellenden Vorrichtung in den zweiten Freiräumen 11 zwischen den Maskenelementen 10 und auf der Oberseite 33 der Maskenelemente 10 abgeschieden wurde. Es deutet sich bereits in diesem Zustand die medizinische Vorrichtung an, wobei hier die Strukturelemente 2 der medizinischen Vorrichtung abgebildet sind.

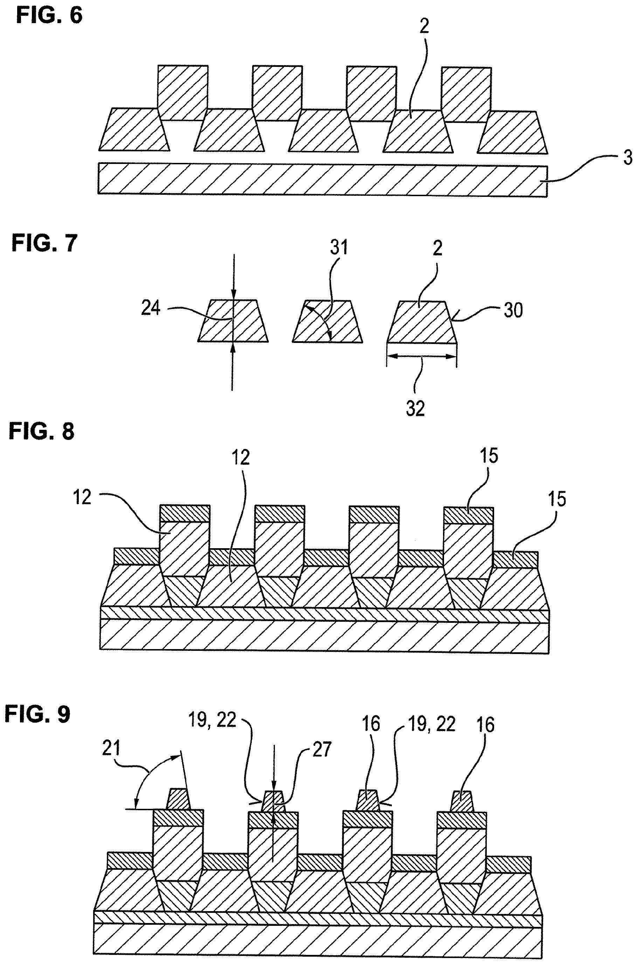

Fig. 6 zeigt nun die medizinische Vorrichtung bzw. die Strukturelemente 2, nachdem das gesamte Opfermaterial 4, 9 abgetragen wurde. Hierfür werden im Ätzverfahren beispielsweise Salpetersäure (HNO₃) verwendet. Es verbleiben die Strukturelemente der medizinischen Vorrichtung.

Fig. 7 zeigt die fertigen Strukturelemente 2 der medizinischen Vorrichtung. Die Strukturelemente 2 können bspw. die Stege einer Gitterstruktur sein. Die Gitterstruktur kann rohrförmig geschlossen sein und bspw. einen Stent oder ein Thrombektomiedevice bilden. Die übrigen Strukturelemente der Gitterstruktur, die entsprechend hergestellt sind, sind in Fig. 7 nicht dargestellt. Die Dimensionen, wie die Höhe 24, der Winkel 31 der Seitenwände und die Breite 32 der Grundfläche der Strukturelemente 2 sind gezeigt.

Die Strukturelemente 2 sind freitragend durch ein PVD-Verfahren hergestellt. Freitragend bedeutet, dass kein anderweitig hergestelltes Basismaterial als Tragstruktur für eine PVD-Beschichtung verwendet wird, sondern das gesamte Strukturelement 2 durch das PVD-Verfahren hergestellt ist.

In den Fig. 8-15 wird das Verfahren in einzelnen ausgewählten Verfahrensschritten gezeigt, mit dem eine Oberflächenprofilierung der medizinischen Vorrichtung möglich ist. Fig. 6 zeigt die medizinische Vorrichtung bzw. die Strukturelemente 2, wie sie noch auf dem Schichtverbund aus Fig. 4 bzw. Fig. 5 angeordnet sind. Wie in Fig. 8 gezeigt, wird auf den Strukturelementen 2 ein weiteres Opfermaterial 15 abgeschieden. Das Opfermaterial 15 bedeckt das gesamte erste Material 12 der herzustellenden Vorrichtung.

Fig. 9 zeigt den nächsten Verfahrensschritt zur Herstellung eines Oberflächenprofils der herzustellenden Strukturelemente 2. Es zeigt eine bereits strukturierte weitere Fotolackschicht 16. Die Seitenflächen 22 der weiteren Fotolackschicht 16 bilden einen Winkel 21 zur weiteren Opfermaterialschicht 15. Die Strukturierung der Fotolackschicht 16 bildet im Wesentlichen ein Positiv der gewünschten Oberflächenprofilierung 34 des herzustellenden Strukturelements. Die strukturierte Fotolackschicht 16 kann bspw. inselförmige Erhebungen bilden, die entlang der Oberfläche der Strukturelemente verteilt angeordnet sind. Diese Form entspricht dem Profil der modifizierten Oberfläche der Strukturelemente 2. Beliebige andere Oberflächenprofile sind durch eine geeignet Strukturierung der Fotolackschicht möglich.

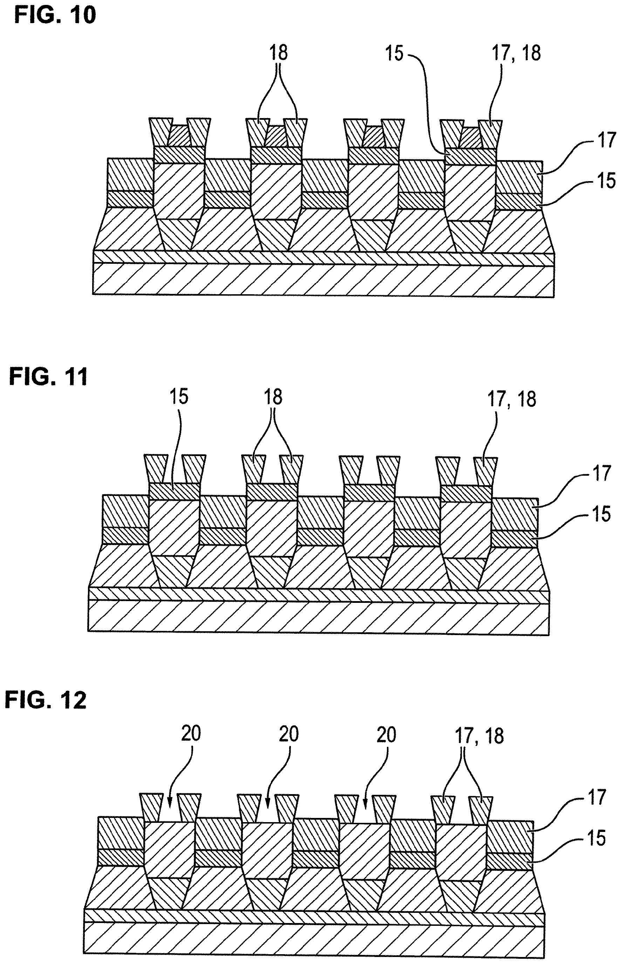

Fig. 10 zeigt den nachfolgenden Verfahrensschritt, in dem eine weitere Opfermaterialschicht 17 aufgetragen wurde. Die Opfermaterialschicht 17 bildet weitere Maskenelemente 18, die um die Fotolackschicht 16 herum gebildet werden. Die Innenkontur der Maskenelemente 18 entspricht der Außenkontur der strukturierten Fotolackschicht.

Fig. 11 zeigt das Verfahren in einem Verfahrensschritt, nachdem die Fotolackschicht 16 entfernt wurde. Anschließend wird, wie in Fig. 12 gezeigt, das Opfermaterial 15 ebenfalls abgetragen, so dass Freiräume 20 zwischen den Maskenelementen 18 entstehen. Die Freiräume 20 sind nach unten durch das erste Material 12 der herzustellenden medizinischen Vorrichtung begrenzt. In horizontaler Richtung, d.h. in paralleler Richtung zum Substrat, sind die Freiräume 20 durch die Maskenelemente 18 begrenzt. Der Winkel der Flanken, den die Maskenelemente 18 zum ersten Material 12 der medizinischen Vorrichtung bilden, wurde durch die strukturierte Fotolackschicht 16 aufgeprägt.

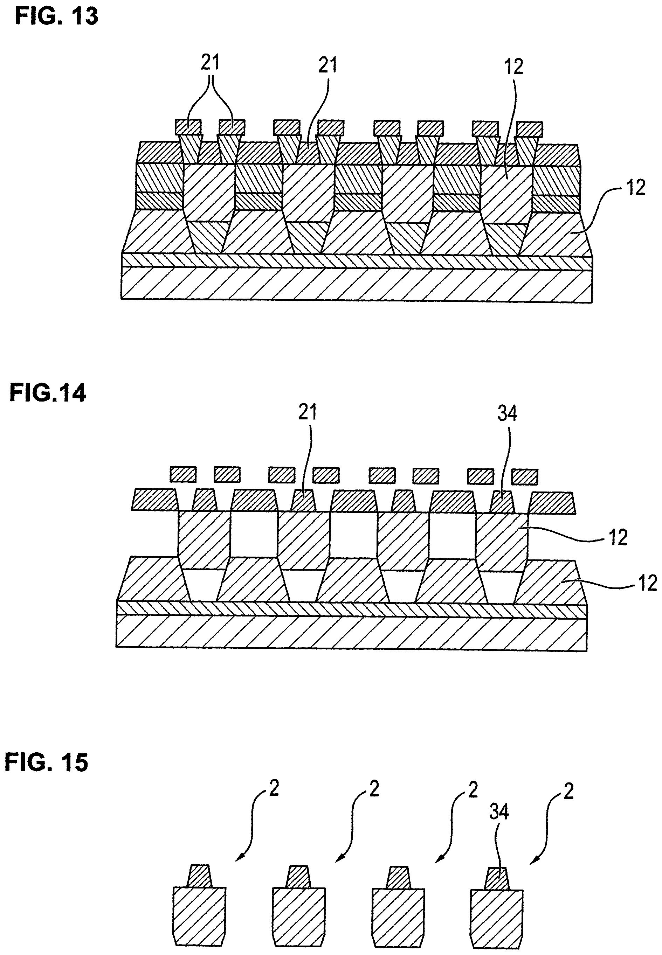

Fig. 13 zeigt das Verfahren in einem Zwischenschritt, nachdem ein zweites Material 21 abgeschieden wurde. Das Material 21 wird gleichmäßig auf die gesamte freiliegende Oberfläche abgeschieden und bildet eine abdeckende Schicht. In dem Bereich, in dem das erste Material 12 der herzustellenden Vorrichtung vor dem Abscheiden des zweiten Materials freiliegt, d.h. in den Freiräumen 20, ist das zweite Material 21 mit dem ersten Material 12 der herzustellenden Vorrichtung stoffschlüssig verbunden.

In einem folgenden Schritt des Verfahrens werden die Opferschichten vollständig entfernt. Dies geschieht mittels eines Ätzverfahrens. Fig. 14 zeigt den Zustand, nachdem die Opferschichten 4, 9, 15, 17 entfernt wurden.

Fig. 15 zeigt die Strukturelemente 2 mit einer Oberflächenprofilierung 34 in einem finalen Zustand.

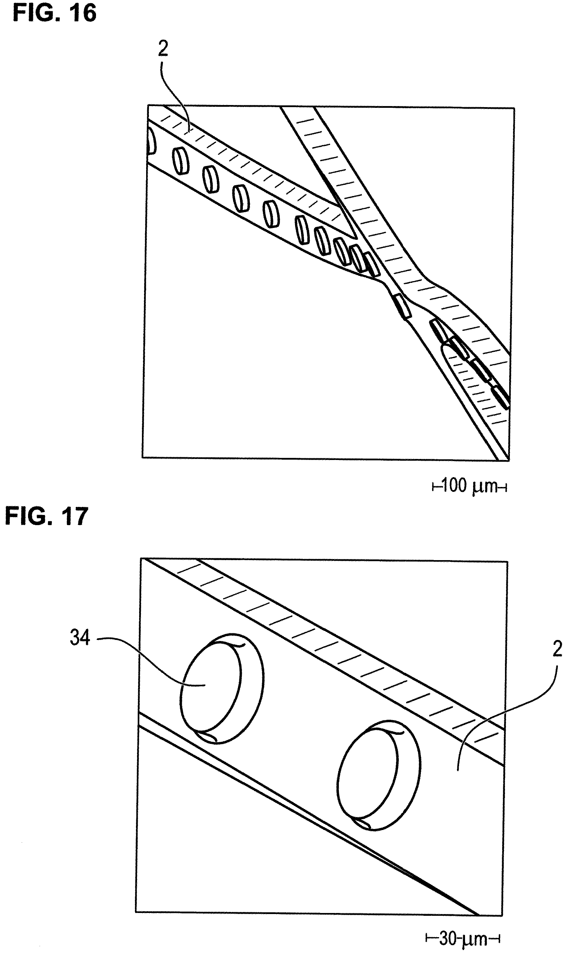

Fig. 16 zeigt ein Beispiel einer medizinischen Vorrichtung, insbesondere die Strukturelemente 2 der medizinischen Vorrichtung mit einer profilierten Oberfläche 34 der Strukturelemente 2. Fig. 17 zeigt eine Vergrößerung der Oberflächenprofilierung 34. Die Profilierung umfasst einzelne Erhebungen, die über die Oberfläche nach Außen vorstehen und zur Verankerung der Gitterstruktur in der Gefäßwand dienen. In der Draufsicht sind die Erhebungen oval. Andere geometrische Formen sind herstellbar. Die Erhebungen sind hintereinander entlang der Strukturelemente, insbesondere Stege, angeordnet. Die Erhebungen können aus demselben oder einem anderen Material als dem Material der Stege gebildet sein.

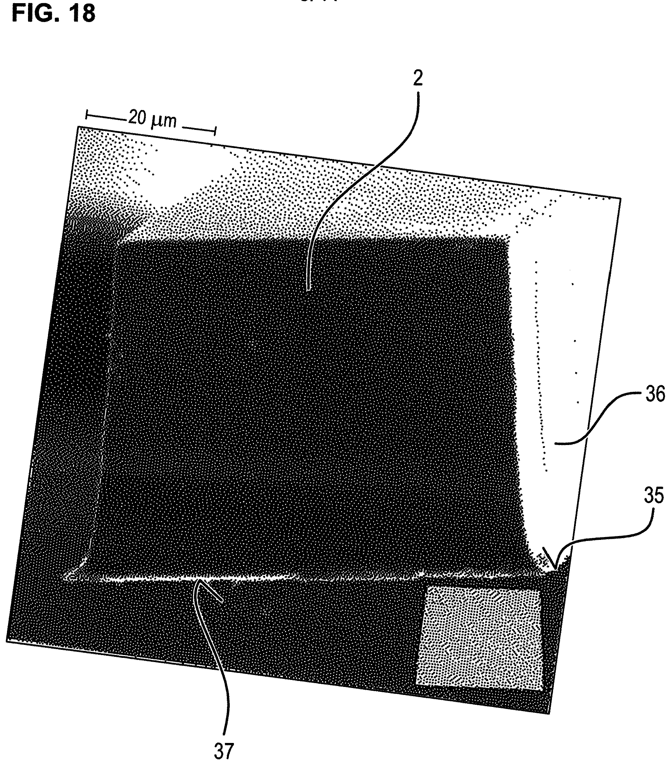

Fig. 18 zeigt eine medizinische Vorrichtung 1 bzw. die Strukturelemente 2 der medizinischen Vorrichtung 1 im Querschnitt. Dieses in Fig. 18 dargestellte Strukturelement ist durch das erfindungsgemäße Verfahren hergestellt. Das Strukturelement weist leicht geneigte Seitenwände 36 auf. Die Seitenwände 36 gehen im unteren Bereich in eine Schneidkante 35 über. Die Schneidkante 35 ist zwischen der Grundfläche 37 des Strukturelements 2 und der geneigten Seitenwand 36 gebildet und weist eine gekrümmte Flanke auf, die in die gerade Fläche der Seitenwand 36 kontinuierlich übergeht. Die Schneidkante 35 ist besonders vorteilhaft, um die medizinische Vorrichtung in einer Gefäßwand zu verankern. Da die Schneidkante 35 die Außenkante des Strukturelements 2 bildet, kann diese zum Ablösen und Entfernen von Ablagerungen und Thromben verwendet werden. Das mit dem erfindungsgemäßen Verfahren hergestellte Strukturelement 2 kann somit ein Steg eines Schneidelements, insbesondere ein scharfkantiger Steg eines Retrievers, d.h. eines Thrombektomie-Devices, sein.

Das Strukturelement 2 gemäß Fig. 18 wurde mit einem Schichtverbund 28 gebildet, dessen Maskenelemente einen relativ großen Abstand 25 voneinander aufweisen. Daher ist das Strukturelement 2 aus Fig. 18 in seiner Grundfläche 37 relativ breit.

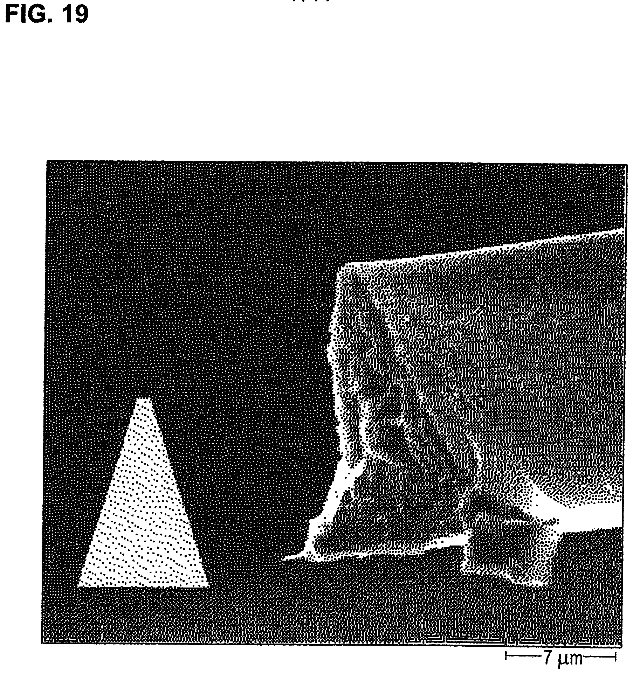

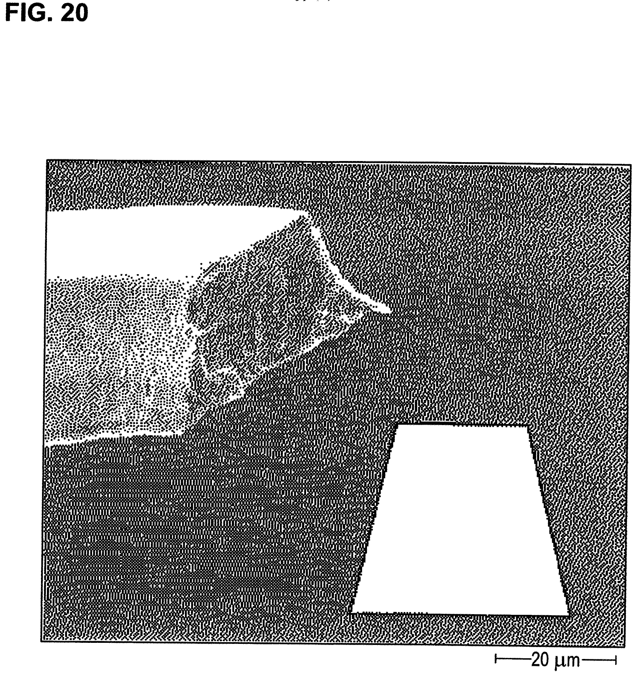

Im Vergleich dazu ist das Strukturelement aus Fig. 19 nahezu dreieckig gestaltet. Es weist eine relativ kleine Grundfläche auf, Seitenwände, die etwas stärker geneigt sind und eine nahezu spitze Oberseite. Fig. 20 zeigt ein Strukturelement 2, das im Querschnitt trapezförmig gebildet ist.

In den Fig. 8 und 21-27 wird ein weiteres Verfahren in einzelnen ausgewählten Verfahrensschritten gezeigt, mit dem eine Oberflächenprofilierung einer Vorrichtung, beispielsweise einer medizinischen Vorrichtung, möglich ist. Fig. 6 zeigt die medizinische Vorrichtung bzw. die Strukturelemente 2, wie sie noch auf dem Schichtverbund aus Fig. 4 bzw. Fig. 5 angeordnet sind. Wie in Fig. 8 gezeigt, wird auf den Strukturelementen 2 ein weiteres Opfermaterial 15 abgeschieden. Das Opfermaterial 15 bedeckt das gesamte erste Material 12 der herzustellenden Vorrichtung.

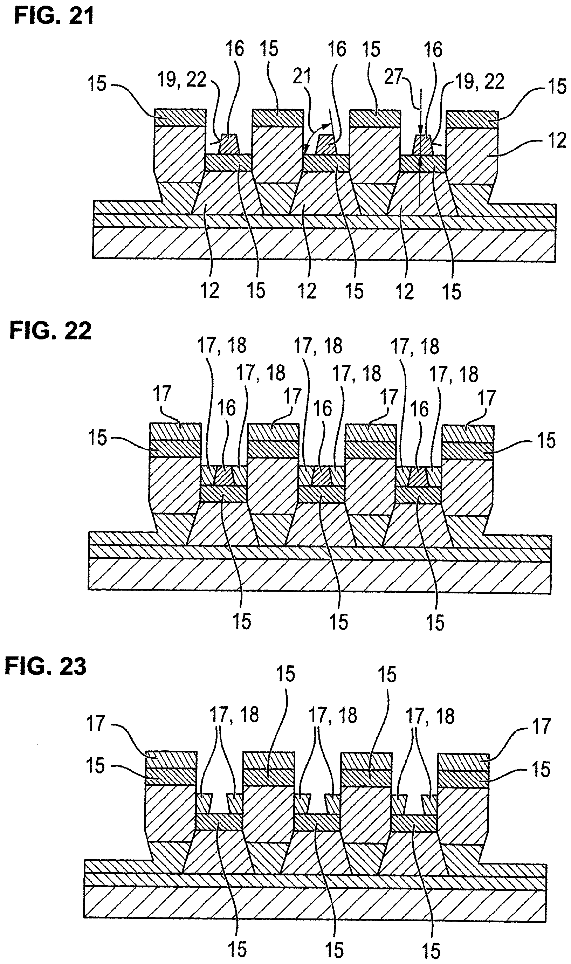

Fig. 21 zeigt den nächsten Verfahrensschritt zur Herstellung eines Oberflächenprofils der herzustellenden Strukturelemente 2. Es zeigt eine bereits strukturierte weitere Fotolackschicht 16. Die Seitenflächen 22 der weiteren Fotolackschicht 16 bilden einen Winkel 21 zur weiteren Opfermaterialschicht 15. Die Strukturierung der Fotolackschicht 16 bzw. die Außenkontur 19 der strukturierten weiteren Fotolackschicht 16 bildet im Wesentlichen ein Positiv der gewünschten Oberflächenprofilierung 34 des herzustellenden Strukturelements. Die strukturierte Fotolackschicht 16 kann bspw. inselförmige Erhebungen bilden, die entlang der Oberfläche der Strukturelemente verteilt angeordnet sind. Im dargestellten Beispiel ist die strukturierte Fotolackschicht 16 in Form von inselförmigen Erhebungen auf den tiefer liegenden Opfermaterialschichten 15 ausgebildet. Diese Form entspricht dem Profil der modifizierten Oberfläche der Strukturelemente 2. Beliebige andere Oberflächenprofile sind durch eine geeignet Strukturierung der Fotolackschicht möglich.

Fig. 22 zeigt den nachfolgenden Verfahrensschritt, in dem eine weitere Opfermaterialschicht 17 aufgetragen wurde. Die Opfermaterialschicht 17 bildet weitere Maskenelemente 18, die um die Fotolackschicht 16 herum gebildet werden. Die Innenkontur der Maskenelemente 18 entspricht der Außenkontur 19 der strukturierten Fotolackschicht.

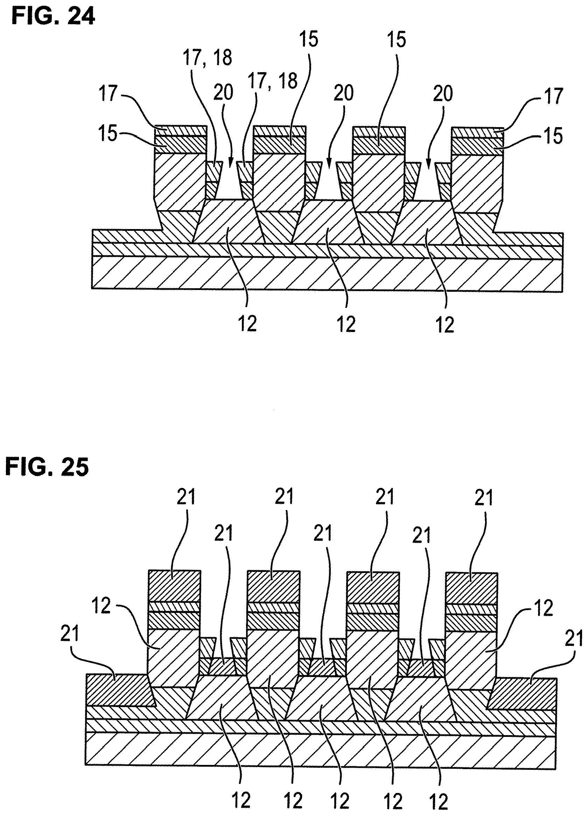

Fig. 23 zeigt das Verfahren in einem Verfahrensschritt, nachdem die Fotolackschicht 16 entfernt wurde. Anschließend wird, wie in Fig. 24 gezeigt, das Opfermaterial 15 ebenfalls abschnittsweise abgetragen, so dass Freiräume 20 zwischen den Maskenelementen 18 entstehen. Das Opfermaterial 15 wird im dargestellen Ausführungsbeispiel in den tiefer liegenden Abschnitten der Opfermaterialschichten 15 abgetragen, nämlich in den Abschnitten, auf denen zuvor die Fotolackschicht 16 aufgetragen war. Die Freiräume 20 sind nach unten durch das erste Material 12 der herzustellenden (medizinischen) Vorrichtung begrenzt. In horizontaler Richtung, d.h. in paralleler Richtung zum Substrat, sind die Freiräume 20 durch die Maskenelemente 18 begrenzt. Der Winkel der Flanken, den die Maskenelemente 18 zum ersten Material 12 der medizinischen Vorrichtung bilden, wurde durch die strukturierte Fotolackschicht 16 aufgeprägt.

Fig. 25 zeigt das Verfahren in einem Zwischenschritt, nachdem ein zweites Material 21 abgeschieden wurde. Das Material 21 wird gleichmäßig auf die freiliegende Oberfläche, insbesondere auf die dargestellte erhöht angeordnete Oberfläche, abgeschieden und bildet eine abdeckende Schicht. In dem Bereich, in dem das erste Material 12 der herzustellenden Vorrichtung vor dem Abscheiden des zweiten Materials freiliegt, d.h. in den Freiräumen 20, ist das zweite Material 21 mit dem ersten Material 12 der herzustellenden Vorrichtung stoffschlüssig verbunden.

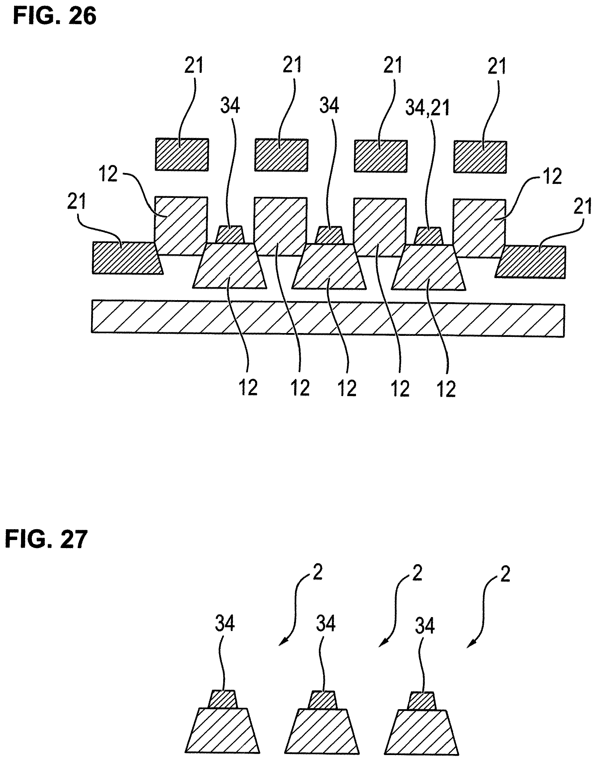

In einem folgenden Schritt des Verfahrens werden die Opferschichten vollständig entfernt. Dies geschieht mittels eines Ätzverfahrens. Fig. 26 zeigt den Zustand, nachdem die Opferschichten 4, 9, 15, 17 entfernt wurden.

Fig. 27 zeigt die Strukturelemente 2 mit einer Oberflächenprofilierung 34 in einem finalen Zustand.

### Bezugszeichenliste

- 1: Medizinische Vorrichtung
- 2: Strukturelement
- 3: Substrat
- 4: Erstes Opfermaterial
- 5: Fotolackschicht
- 6: Erste Freiräume
- 7: Seitenflächen der Fotolackschicht 5
- 8: Winkel zwischen Seitenflächen und Substrat
- 9: Opfermaterial
- 10: Erste Maskenelemente
- 11: Zweite Freiräume
- 12: Erstes Material der herzustellenden Vorrichtung
- 13: Innenkontur der ersten Freiräume 6
- 14: Flanken der ersten Maskenelemente 10
- 15: Weitere Opfermaterialschicht
- 16: Weitere Fotolackschicht
- 17: Opfermaterial
- 18: Maskenelement
- 19: Außenkontur der strukturierten weiteren Fotolackschicht
- 20: Dritter Freiraum
- 21: Winkelflächen zwischen Seitenflächen 22 und weiteren Fotolackschicht 16
- 22: Seitenflächen der weiteren Fotolackschicht 16
- 23: Höhe der Maskenelemente
- 24: Höhe der herzustellenden Strukturelemente 2
- 25: Abstand der ersten Maskenelemente 10
- 26, 27: Höhe der Fotolackschicht 5, 16
- 28: Schichtverbund
- 29: Winkel der Flanken
- 30: Seitenwände der herzustellenden Vorrichtung 1
- 31: Winkel der Seitenwände 30 der herzustellenden Vorrichtung 1
- 32: Breite eines Strukturelements 2
- 33: Oberseite der Maskenelemente
- 34: Oberflächenprofilierung
- 35: Schneidkante
- 36: Seitenwände eines Strukturelements 2
- 37: Grundfläche eines Strukturelements 2

## Patentansprüche

1. Verfahren zur Herstellung einer medizinischen Vorrichtung (1) mit Strukturelementen (2), umfassend folgende Verfahrensschritte:
- Bereitstellen eines Substrates (3);
- Abscheiden einer Schicht aus einem Opfermaterial (4) auf dem Substrat (3);
- Auftragen einer Fotolackschicht (5) auf die Opfermaterialschicht (4),
- Strukturieren der Fotolackschicht (5) gemäß der Form der herzustellenden Strukturelemente (2), so dass erste Freiräume (6) gebildet werden, die auf der Substrat (3) abgewandten Seite offen sind und durch Seitenflächen (7) der Fotolackschicht (5) begrenzt werden, wobei ein Winkel (8), der 45° bis kleiner als 90° beträgt, zwischen den Seitenflächen (7) und dem Substrat (3) eingestellt wird;
- Abscheiden von Opfermaterial (9), so dass in den ersten Freiräumen (6) erste Maskenelemente (10) aus Opfermaterial (9) gebildet werden, die an die Innenkontur (13) der ersten Freiräume (6) angepasst sind;
- Entfernen der Fotolackschicht (5), so dass zweite Freiräume (11) zwischen den ersten Maskenelementen (10) gebildet werden;
- Abscheiden, insbesondere physikalisches Gasphasenabscheiden, eines ersten Materials (12) der herzustellenden Vorrichtung (1) in den zweiten Freiräumen (11) und auf der Oberseite (33) der ersten Maskenelemente (10); und
- Abtragen des Opfermaterials (4, 9) und/oder Durchführen weiterer Prozessschritte.

2. Verfahren zur Herstellung einer Vorrichtung mit Strukturelementen (2), insbesondere zur Herstellung von Aktoren und/oder Mikropumpen und/oder linearen Aktoren im Bereich der Mikrosystemtechnik und/oder Aktoren für minimal invasive Instrumente und/oder Mikroaktoren und/oder flexible Mikroantennen, umfassend folgende Verfahrensschritte:
- Bereitstellen eines Substrates (3);
- Abscheiden einer Schicht aus einem Opfermaterial (4) auf dem Substrat (3);
- Auftragen einer Fotolackschicht (5) auf die Opfermaterialschicht (4),
- Strukturieren der Fotolackschicht (5) gemäß der Form der herzustellenden Strukturelemente (2), so dass erste Freiräume (6) gebildet werden, die auf der Substrat (3) abgewandten Seite offen sind und durch Seitenflächen (7) der Fotolackschicht (5) begrenzt werden, wobei ein Winkel (8), der 45° bis kleiner als 90° beträgt, zwischen den Seitenflächen (7) und dem Substrat (3) eingestellt wird;
- Abscheiden von Opfermaterial (9), so dass in den ersten Freiräumen (6) erste Maskenelemente (10) aus Opfermaterial (9) gebildet werden, die an die Innenkontur (13) der ersten Freiräume (6) angepasst sind;
- Entfernen der Fotolackschicht (5), so dass zweite Freiräume (11) zwischen den ersten Maskenelementen (10) gebildet werden;
- Abscheiden, insbesondere physikalisches Gasphasenabscheiden, eines ersten Materials (12) der herzustellenden Vorrichtung (1) in den zweiten Freiräumen (11) und auf der Oberseite (33) der ersten Maskenelemente (10); und
- Abtragen des Opfermaterials (4, 9) und/oder Durchführen weiterer Prozessschritte.

3. Verfahren nach Anspruch 1 oder 2,
**dadurch gekennzeichnet, dass**
die Seitenflächen (7) jeweils eines Freiraumes (6, 11) trichterförmig geöffnet sind.

4. Verfahren nach einem der vorhergehenden Ansprüche,
**dadurch gekennzeichnet, dass**
die ersten Maskenelemente (10) Flanken (14) aufweisen, die entsprechend dem Winkel (8) der Seitenflächen (7) geneigt sind.

5. Verfahren nach einem der vorhergehenden Ansprüche,
**dadurch gekennzeichnet, dass**
die Fotolackschicht (5) derart strukturiert wird, dass der Verlauf stegartiger Strukturelemente (2), insbesondere der Verlauf der Stege einer Gitterstruktur, abgebildet wird.

6. Verfahren nach einem der vorhergehenden Ansprüche umfassend folgende weitere Prozessschritte:
- Optionales Abscheiden einer weiteren Opfermaterialschicht (15) auf dem Material (12) der herzustellenden Vorrichtung;
- Auftragen einer weiteren Fotolackschicht (16) und Strukturieren der weiteren Fotolackschicht (16) gemäß der Form einer Oberflächenprofilierung (34) der herzustellenden Strukturelemente (2);
- Abscheiden von Opfermaterial (17), so dass wenigstens ein weiteres zweites Maskenelement (18) gebildet wird, das an die Außenkontur (19) der strukturierten weiteren Fotolackschicht (16) angepasst ist;
- Entfernen der Fotolackschicht (16) und der optionalen weiteren Opfermaterialschicht (15), so dass ein dritter Freiraum (20) gebildet wird, der das Material (12) der herzustellenden Vorrichtung freilegt und von dem weiteren Maskenelement (18) oder von mehreren weiteren Maskenelementen (18) begrenzt wird;
- Abscheiden, insbesondere physikalisches Gasphasenabscheiden, des ersten oder eines zweiten Materials (12, 21) der herzustellenden Vorrichtung im dritten Freiraum (20); und
- Abtragen des Opfermaterials (15, 17).

7. Verfahren nach Anspruch 6,
**dadurch gekennzeichnet, dass**
während des Strukturierens der weiteren Fotolackschicht (16) ein Winkel (21) zwischen den Seitenflächen (22) der weiteren Fotolackschicht (16) und der weiteren Opfermaterialschicht (15) eingestellt wird.

8. Verfahren nach einem der vorhergehenden Ansprüche,
**dadurch gekennzeichnet, dass**
die Opfermaterialschicht, insbesondere die Opfermaterialschichten (4, 9, 15, 17), elektrisch leitend ist und die Maskenelemente (10, 18) elektrochemisch auf den elektrisch leitenden Opfermaterialschichten (4, 15) abgeschieden werden.

9. Verfahren nach einem der vorhergehenden Ansprüche,
**dadurch gekennzeichnet, dass**
das Material (12, 21) der herzustellenden Vorrichtung durch Sputtern abgeschieden wird.

10. Verfahren nach einem der vorhergehenden Ansprüche,
**dadurch gekennzeichnet, dass**
die Höhe (23) der Maskenelemente (10, 18), insbesondere der ersten und zweiten Maskenelemente, zur Beeinflussung der maximalen Höhe (24) der herzustellenden Strukturelemente (2) eingestellt wird.

11. Verfahren nach einem der vorhergehenden Ansprüche,
**dadurch gekennzeichnet, dass**
der Abstand (15) der ersten Maskenelemente (10) zur Beeinflussung der Breite der herzustellenden Strukturelemente eingestellt wird.

12. Verfahren nach einem der vorhergehenden Ansprüche,
**dadurch gekennzeichnet, dass**
die ersten und/oder zweiten Maskenelemente (10, 18) eine Höhe (23) von 2 bis 100 pm aufweisen.

13. Verfahren nach einem der vorhergehenden Ansprüche,
**dadurch gekennzeichnet, dass**
das erste Material (12) der medizinischen Vorrichtung (1) oder der Vorrichtung mit Strukturelementen und/oder
das zweite Material (21) der medizinischen Vorrichtung (1) oder der Vorrichtung mit Strukturelementen ein Formgedächtnismaterial, insbesondere NiTi, und/oder ein Schwermetall, insbesondere Tantal, und/oder ein elektrisch leitendes Material umfasst.

14. Verfahren zum Modifizieren der Oberfläche einer medizinischen Vorrichtung (1) mit Strukturelementen (2), umfassend folgende Verfahrensschritte:
- Bereitstellen der medizinischen Vorrichtung (1) mit Strukturelementen (2);
- Abscheiden einer Opfermaterialschicht (15) auf dem Material (12) der Vorrichtung (1) im Bereich der zu modifizierenden Oberfläche;
- Auftragen einer Fotolackschicht (16) und Strukturieren der Fotolackschicht (16) gemäß der Form der herzustellenden Oberflächenprofilierung (34) der Strukturelemente (2);
- Abscheiden von Opfermaterial (17), so dass wenigstens ein Maskenelement (18) gebildet wird, das an die Außenkontur (19) der strukturierten Fotolackschicht (16) angepasst ist;
- Entfernen der Fotolackschicht (16) und der optionalen Opfermaterialschicht (15), so dass ein Freiraum (20) gebildet wird, der das Material (12) der herzustellenden Vorrichtung (1) freilegt und von dem weiteren Maskenelement (18) oder von mehreren weiteren Maskenelementen (18) begrenzt wird;
- Abscheiden, insbesondere physikalisches Gasphasenabscheiden, des Materials (12, 21) der Vorrichtung (1) und/oder eines anderen Materials im Freiraum (20); und
- Abtragen des Opfermaterials (15, 17).

15. Verfahren zum Modifizieren der Oberfläche einer Vorrichtung mit Strukturelementen (2), insbesondere einer Oberfläche von Aktoren und/oder Mikropumpen und/oder lineare Aktoren im Bereich der Mikrosystemtechnik und/oder Aktoren für minimal invasive Instrumente, Mikroaktoren und/oder flexiblen Mikorantennen, umfassend folgende Verfahrensschritte:
- Bereitstellen der Vorrichtung mit Strukturelementen (2);
- Abscheiden einer Opfermaterialschicht (15) auf dem Material (12) der Vorrichtung im Bereich der zu modifizierenden Oberfläche;
- Auftragen einer Fotolackschicht (16) und Strukturieren der Fotolackschicht (16) gemäß der Form der herzustellenden Oberflächenprofilierung (34) der Strukturelemente (2);
- Abscheiden von Opfermaterial (17), so dass wenigstens ein Maskenelement (18) gebildet wird, das an die Außenkontur (19) der strukturierten Fotolackschicht (16) angepasst ist;
- Entfernen der Fotolackschicht (16) und der optionalen Opfermaterialschicht (15), so dass ein Freiraum (20) gebildet wird, der das Material (12) der herzustellenden Vorrichtung freilegt und von dem weiteren Maskenelement (18) oder von mehreren weiteren Maskenelementen (18) begrenzt wird;
- Abscheiden, insbesondere physikalisches Gasphasenabscheiden, des Materials (12, 21) der Vorrichtung und/oder eines anderen Materials im Freiraum (20); und
- Abtragen des Opfermaterials (15, 17).

16. Medizinische Vorrichtung, die durch ein Verfahren nach einem der vorhergehenden Ansprüche hergestellt ist,
**dadurch gekennzeichnet, dass**
die Strukturelemente (2) der medizinischen Vorrichtung wenigstens eine geneigte Seitenwand (36) mit einer Schneidkante (35) aufweist, die zwischen der Seitenwand (36) und einer Grundfläche (37) des Strukturelements (2) gebildet ist, wobei die Schneidkante (35) über die Fläche der Seitenwand (36) vorsteht und eine gekrümmte Flanke aufweist, die kontinuierlich in die Fläche der Seitenwand (36) übergeht.

17. Schichtverbund (28) mit einem Substrat (3) und einer Opfermaterialschicht (4) für die Herstellung einer medizinischen Vorrichtung (1) mit Strukturelementen (2), wobei die Opfermaterialschicht (4) auf dem Substrat (3) angeordnet ist und auf der Opfermaterialschicht (4) Maskenelemente (10) aus einem Opfermaterial (9) gebildet sind, deren Flanken einen Winkel (29) aufweisen, der komplementär zum Winkel (31) der Seitenwände (30) der herzustellenden Vorrichtung (1) gebildet ist, wobei der Abstand (25) zwischen den Maskenelementen der Breite (32) eines Strukturelements (2) der herzustellenden Vorrichtung (1) entspricht.

18. Schichtverbund (28) mit einem Substrat (3) und einer Opfermaterialschicht (4) für die Herstellung einer Vorrichtung mit Strukturelementen (2), insbesondere für die Herstellung von Aktoren und/oder Mikropumpen und/oder linearen Aktoren im Bereich der Mikrosystemtechnik und/oder Aktoren für minimal invasive Instrumente und/oder Mikroaktoren und/oder flexiblen Mikroantennen, wobei die Opfermaterialschicht (4) auf dem Substrat (3) angeordnet ist und auf der Opfermaterialschicht (4) Maskenelemente (10) aus einem Opfermaterial (9) gebildet sind, deren Flanken einen Winkel (29) aufweisen, der komplementär zum Winkel (31) der Seitenwände (30) der herzustellenden Vorrichtung gebildet ist, wobei der Abstand (25) zwischen den Maskenelementen der Breite (32) eines Strukturelements (2) der herzustellenden Vorrichtung entspricht.

## Claims

1. Method for producing a medical device (1) with structure elements (2), comprising the following method steps:
- providing a substrate (3);
- depositing a layer of a sacrificial material (4) on the substrate (3);
- applying a photoresist layer (5) on the sacrificial material layer (4),
- structuring the photoresist layer (5) according to the shape of the structure elements (2) to be produced, so that first free spaces (6) are formed which are open on the side facing away from the substrate (3) and are delimited by side surfaces (7) of the photoresist layer (5), wherein an angle (8) of 45° to less than 90° is set between the side surfaces (7) and the substrate (3);
- depositing sacrificial material (9) such that first mask elements (10) of sacrificial material (9) are formed in the first free spaces (6) and are adapted to the inner contour (13) of said first free spaces (6);
- removing the photoresist layer (5) so that second free spaces (11) are formed between the first mask elements (10);
- depositing, particularly by physical vapour deposition, a first material (12) of the device to be produced (1) in the second free spaces (11) and on the upper side (33) of the first mask elements (10); and
- removing the sacrificial material (4, 9) and/or performing further process steps.

2. Method for producing a device with structure elements (2), particularly for producing actuators and/or micropumps and/or linear actuators in the field of microsystems technology and/or actuators for minimally invasive instruments and/or microactuators and/or flexible microantennas, comprising the following method steps:
- providing a substrate (3);
- depositing a layer of a sacrificial material (4) on the substrate (3);
- applying a photoresist layer (5) on the sacrificial material layer (4),
- structuring the photoresist layer (5) according to the shape of the structure elements (2) to be produced, so that first free spaces (6) are formed which are open on the side facing away from the substrate (3) and are delimited by side surfaces (7) of the photoresist layer (5), wherein an angle (8) of 45° to less than 90° is set between the side surfaces (7) and the substrate (3);
- depositing sacrificial material (9) such that first mask elements (10) of sacrificial material (9) are formed in the first free spaces (6) and are adapted to the inner contour (13) of said first free spaces (6);
- removing the photoresist layer (5) so that second free spaces (11) are formed between the first mask elements (10);
- depositing, particularly by physical vapour deposition, a first material (12) of the device to be produced (1) in the second free spaces (11) and on the upper side (33) of the first mask elements (10); and
- removing the sacrificial material (4, 9) and/or performing further process steps.

3. Method according to Claim 1 or 2, **characterized in that** the side surfaces (7) of each free space (6, 11) are open in the form of a funnel.

4. Method according to any one of the preceding claims, **characterized in that** the first mask elements (10) have flanks (14) that are inclined correspondingly to the angle (8) of the side surfaces (7).

5. Method according to any one of the preceding claims, **characterized in that** the photoresist layer (5) is structured in such manner that the course of web-like structure elements (2), particularly the course of the webs of a lattice structure, is reproduced.

6. Method according to any one of the preceding claims comprising the following further process steps:
- optionally depositing a further sacrificial material layer (15) on the material (12) of the device to be produced;
- applying a further photoresist layer (16) and structuring the further photoresist layer (16) according to the shape of a surface profile (34) of the structure elements (2) to be produced;
- depositing sacrificial material (17) in such manner that at least one further second mask element (18) is formed which is adapted to the outer contour (19) of the structured further photoresist layer (16);
- removing the photoresist layer (16) and the optional further sacrificial material layer (15), so that a third free space (20) is formed which exposes the material (12) of the device to be produced and is delimited by the further mask element (18) or by several further mask elements (18);
- depositing, particularly by physical vapour deposition, the first or a second material (12, 21) of the device to be produced in the third free space (20); and
- removing the sacrificial material (15, 17).

7. Method according to claim 6, **characterized in that** an angle (21) between the side surfaces (22) of the further photoresist layer (16) and the further sacrificial material layer (15) is set during the structuring of the further photoresist layer (16).

8. Method according to any one of the preceding claims, **characterized in that** the sacrificial material layer, in particular the sacrificial material layers (4, 9, 15, 17) is electrically conductive, and the mask elements (10, 18) are deposited electrochemically on the electrically conductive sacrificial material layers (4, 15).

9. Method according to any one of the preceding claims, **characterized in that** the material (12, 21) of the device to be produced is deposited by sputtering.

10. Method according to any one of the preceding claims, **characterized in that** the height (23) of the mask elements (10, 18), particularly the first and second mask elements is adjusted to influence the maximum height (24) of the structure elements (2) that are to be produced.

11. Method according to any one of the preceding claims, **characterized in that** the distance (15) between the first mask elements (10) is adjusted to influence the width of the structure elements that are to be produced.

12. Method according to any one of the preceding claims, **characterized in that** the first and/or second mask elements (10, 18) have a height (23) from 2 to 100 pm.

13. Method according to any one of the preceding claims, **characterized in that** the first material (12) of the medical device (1) or of the device with structure elements and/or the second material (21) of the medical device (1) or of the device with structure elements comprises a shape memory material, particularly NiTi, and/or a heavy metal, particularly tantalum, and/or an electrically conductive material.

14. Method for modifying the surface of a medical device (1) with structural elements (2), comprising the following method steps:
- providing the medical device (1) with structure elements (2);
- depositing a sacrificial material layer (15) on the material (12) of the device (1) in the region of the surface that is to be modified;
- applying a photoresist layer (16) and structuring the photoresist layer (16) according to the shape of the surface profile (34) of the structure elements (2) that is to be produced;
- depositing sacrificial material (17) in such manner that at least one mask element (18) is formed which is adapted to the outer contour (19) of the structured photoresist layer (16);
- removing the photoresist layer (16) and the optional sacrificial material layer (15) so that a free space (20) is formed which exposes the material (12) of the device (1) that is to be produced and is delimited by the further mask element (18) or by several further mask elements (18);
- depositing, particularly by physical vapour deposition, the material (12, 21) of the device (1) and/or another material in the free space (20); and
- removing the sacrificial material (15, 17).

15. Method for modifying the surface of a device having structure elements (2), particularly a surface of actuators and/or micropumps and/or linear actuators in the field of microsystems technology and/or actuators for minimally invasive instruments, microactuators and/or flexible microantennas, comprising the following method steps:
- providing the device with structure elements (2);
- depositing a sacrificial material layer (15) on the material (12) of the device in the region of the surface that is to be modified;
- applying a photoresist layer (16) and structuring the photoresist layer (16) according to the shape of the surface profile (34) of the structure elements (2) that is to be produced;
- depositing sacrificial material (17) in such manner that at least one mask element (18) is formed which is adapted to the outer contour (19) of the structured photoresist layer (16);
- removing the photoresist layer (16) and the optional sacrificial material layer (15) so that a free space (20) is formed which exposes the material (12) of the device that is to be produced and is delimited by the further mask element (18) or by several further mask elements (18);
- depositing, particularly by physical vapour deposition, the material (12, 21) of the device and/or another material in the free space (20); and
- removing the sacrificial material (15, 17).

16. Medical device produced by a method according to any one of the preceding claims, **characterized, in that** the structure elements (2) of the medical device have at least one inclined side wall (36) with a cutting edge (35) which is formed between the side wall (36) and a base surface (37) of the structure element (2), wherein the cutting edge (35) protrudes beyond the surface of the side wall (36) and has a curved flank which transitions continuously into the surface of the side wall (36).

17. Laminated composite (28) comprising a substrate (3) and a sacrificial material layer (4) for producing a medical device (1) with structure elements (2), wherein the sacrificial material layer (4) is disposed on the substrate (3) and mask elements (10) are formed on the sacrificial material layer (4) from a sacrificial material (9), the flanks of which mask elements form an angle (29) that is complementary to the angle (31) of the side walls (30) of the device (1) that is to be produced, wherein the distance (25) between the mask elements corresponds to the width (32) of a structure element (2) of the device to be produced (1).

18. Laminated composite (28) comprising a substrate (3) and a sacrificial material layer (4) for producing a device with structure elements (2), particularly for producing actuators and/or micropumps and/or linear actuators in the field of microsystems technology and/or actuators for minimally invasive instruments and/or microactuators and/or flexible microantennas, wherein the sacrificial material layer (4) is disposed on the substrate (3) and mask elements (10) are formed on the sacrificial material layer (4) from a sacrificial material (9), the flanks of which mask elements form an angle (29) that is complementary to the angle (31) of the side walls (30) of the device that is to be produced, wherein the distance (25) between the mask elements corresponds to the width (32) of a structure element (2) of the device to be produced.

## Revendications

1. Procédé de production d'un dispositif médical (1) muni d'éléments de structure (2), comprenant les étapes de procédé suivantes :
- fourniture d'un substrat (3) ;
- séparation d'une couche dans un matériau sacrificiel (4) sur le substrat (3) ;
- application d'une couche de photo-résine (5) sur la couche de matériau sacrificiel (4),
- structuration de la couche de photo-résine (5) selon la forme des éléments de structure (2) à produire, de sorte que des premiers espaces libres (6) soient formés qui sont ouverts sur le côté détourné du substrat (3) et sont délimités par des faces latérales (7) de la couche de photo-résine (5), dans lequel un angle (8), qui fait de 45° à moins de 90° est créé entre les faces latérales (7) et le substrat (3) ;
- séparation du matériau sacrificiel (9) de sorte que dans les premiers espaces libres (6), des premiers éléments de masque (10) en matériau sacrificiel (9) soient formés qui sont adaptés au contour intérieur (13) des premiers espaces libres (6) ;
- enlèvement de la couche de photo-résine (5) de façon à ce que des deuxièmes espaces libres (11) soient formés entre les premiers éléments de masque (10) ;
- séparation, en particulier séparation en phase gazeuse physique, d'un premier matériau (12) du dispositif (1) à produire dans les deuxièmes espaces libres (11) et sur la face supérieure (33) des premiers éléments de masque (10) ; et
- décapage du matériau sacrificiel (4, 9) et/ou exécution d'autres étapes de procédé.

2. Procédé de production d'un dispositif muni d'éléments de structure (2), en particulier de production d'actionneurs et/ou de micro-pompes et/ou d'actionneurs linéaires dans le domaine de la technique de micro-systèmes et/ou d'actionneurs pour des instruments invasifs minimaux et/ou des micro-actionneurs et/ou des micro-antennes flexibles, comprenant les étapes de procédé suivantes :
- fourniture d'un substrat (3) ;
- séparation d'une couche dans un matériau sacrificiel (4) sur le substrat (3) ;
- application d'une couche de photo-résine (5) sur la couche de matériau sacrificiel (4),
- structuration de la couche de photo-résine (5) selon la forme des éléments de structure (2) à produire, de sorte que des premiers espaces libres (6) soient formés qui sont ouverts sur le côté détourné du substrat (3) et sont délimités par des faces latérales (7) de la couche de photo-résine (5), dans lequel un angle (8), qui fait de 45° à moins de 90° est créé entre les faces latérales (7) et le substrat (3) ;
- séparation du matériau sacrificiel (9) de sorte que dans les premiers espaces libres (6), des premiers éléments de masque (10) en matériau sacrificiel (9) soient formés qui sont adaptés au contour intérieur (13) des premiers espaces libres (6) ;
- enlèvement de la couche de photo-résine (5) de façon à ce que des deuxièmes espaces libres (11) soient formés entre les premiers éléments de masque (10) ;
- séparation, en particulier séparation en phase gazeuse physique, d'un premier matériau (12) du dispositif (1) à produire dans les deuxièmes espaces libres (11) et sur la face supérieure (33) des premiers éléments de masque (10) ; et
- décapage du matériau sacrificiel (4, 9) et/ou exécution d'autres étapes de procédé.

3. Procédé selon la revendication 1 ou 2, **caractérisé en ce que** les faces latérales (7) d'un espace libre (6, 11) respectif sont en forme de trémie.

4. Procédé selon l'une des revendications précédentes, **caractérisé en ce que** les premier éléments de masque (10) présentent des flancs (14) qui sont inclinés conformément à l'angle (8) des faces latérales (7).

5. Procédé selon l'une des revendications précédentes, **caractérisé en ce que** la couche de photo-résine (5) est ainsi structurée que le tracé d'éléments de structure (2) en forme de traverse, en particulier le tracé des traverses d'une structure maillée, est formé.

6. Procédé selon l'une des revendications précédentes, comprenant les autres étapes de procédé suivantes :
- séparation en option d'une couche de matériau sacrificiel supplémentaire (15) sur le matériau (12) du dispositif à produire ;
- application d'une couche de photo-résine supplémentaire (16) et structuration de la couche de photo-résine supplémentaire (16) selon la forme d'un profilé surfacique (34) des éléments de structure (2) à produire ;
- séparation de matériau sacrificiel (7) de façon à ce qu'au moins un deuxième élément de masque supplémentaire (18) soit formé qui est adapté au contour extérieur (19) de la couche de photo-résine supplémentaire (16) structurée ;
- enlèvement de la couche de photo-résine (16) et de la couche de matériau sacrificiel supplémentaire en option (15) de sorte qu'un troisième espace libre (20) soit formé qui libère le matériau (12) du dispositif à produire et est délimité par l'élément de masque supplémentaire (18) ou par plusieurs éléments de masque supplémentaires (18) ;
- séparation, en particulier séparation en phase gazeuse physique, du premier ou d'un deuxième matériau (12, 21) du dispositif à produire dans le troisième espace libre (20) ; et
- décapage du matériau sacrificiel (15, 17).

7. Procédé selon la revendication 6, **caractérisé en ce que** pendant la structuration de la couche de photo-résine supplémentaire (16), un angle (21) entre les faces latérales (22) de la couche de photo-résine supplémentaire (16) et de la couche de matériau sacrificiel supplémentaire (15) est créé.

8. Procédé selon l'une des revendications précédentes, **caractérisé en ce que** la couche de matériau sacrificiel, en particulier les couches de matériau sacrificiel (4, 9, 15, 17), est conductrice(s) d'électricité et les éléments de masque (10, 18) sont séparés par électrochimie sur les couches de matériau sacrificiel (4, 15) conductrices d'électricité.

9. Procédé selon l'une des revendications précédentes, **caractérisé en ce que** le matériau (12, 21) du dispositif à fabriquer est séparé par pulvérisation cathodique.

10. Procédé selon l'une des revendications précédentes, **caractérisé en ce que** la hauteur (23) des éléments de masque (10, 18), en particulier des premier et deuxième éléments de masque, est réglée pour avoir une influence sur la hauteur maximale (24) des éléments de structure (2) à produire.

11. Procédé selon l'une des revendications précédentes, **caractérisé en ce que** l'écart (15) des premiers éléments de masque (10) est réglé pour avoir une influence sur la largeur des éléments de structure à produire.

12. Procédé selon l'une des revendications précédentes, **caractérisé en ce que** les premier et/ou les deuxième éléments de masque (10, 18) présentent une hauteur (23) de 2 à 100 pm.

13. Procédé selon l'une des revendications précédentes, **caractérisé en ce que** le premier matériau (12) du dispositif médical (1) ou du dispositif muni d'éléments de structure et/ou le deuxième matériau (21) du dispositif médical (1) ou du dispositif muni d'éléments de structure comprend un matériau à mémoire de forme, en particulier du NiTi, et/ou un métal lourd, en particulier du tantale, et/ou un matériau conducteur électrique.

14. Procédé de modification d'une surface d'un dispositif médical (1) muni d'éléments de structure (2), comprenant les étapes de procédé suivantes :
- fourniture du dispositif médical (1) muni d'éléments de structure (2) ;
- séparation d'une couche de matériau sacrificiel (15) sur le matériau (12) du dispositif (1) au niveau de la surface à modifier ;
- application d'une couche de photo-résine (16) et structuration de la couche de photo-résine (16) selon la forme du profilé surfacique (34) à produire des éléments de structure (2) ;
- séparation de matériau sacrificiel (17) de façon à ce qu'au moins un élément de masque (18) soit formé qui est adapté au contour extérieur (19) de la couche de photo-résine (16) structurée ;
- enlèvement de la couche de photo-résine (16) et de la couche de matériau sacrificiel en option (15) de sorte qu'un espace libre (20) soit formé qui libère le matériau (12) du dispositif (1) à produire et est délimité par l'élément de masque supplémentaire (18) ou par plusieurs éléments de masque supplémentaires (18) ;
- séparation, en particulier séparation en phase gazeuse physique, du matériau (12, 21) du dispositif (1) et/ou d'un autre matériau dans l'espace libre (20) ; et
- décapage du matériau sacrificiel (15, 17).

15. Procédé de modification de surface d'un dispositif muni d'éléments de structure (2), en particulier d'une surface d'actionneurs et/ou de micro-pompes et/ou d'actionneurs linéaires dans le domaine de la technique de micro-systèmes et/ou d'actionneurs pour des instruments invasifs minimaux et/ou des micro-actionneurs et/ou des micro-antennes flexibles, comprenant les étapes de procédé suivantes :
- fourniture du dispositif muni d'éléments de structure (2) ;
- séparation d'une couche de matériau sacrificiel (15) sur le matériau (12) du dispositif au niveau de la surface à modifier ;
- application d'une couche de photo-résine (16) et structuration de la couche de photo-résine (16) selon la forme du profilé surfacique (34) à produire des éléments de structure (2) ;
- séparation de matériau sacrificiel (17) de façon à ce qu'au moins un élément de masque (18) soit formé qui est adapté au contour extérieur (19) de la couche de photo-résine (16) structurée ;
- enlèvement de la couche de photo-résine (16) et de la couche de matériau sacrificiel en option (15) de sorte qu'un espace libre (20) soit formé qui libère le matériau (12) du dispositif à produire et est délimité par l'élément de masque supplémentaire (18) ou par plusieurs éléments de masque supplémentaires (18) ;
- séparation, en particulier séparation en phase gazeuse physique, du matériau (12, 21) du dispositif et/ou d'un autre matériau dans l'espace libre (20) ; et
- décapage du matériau sacrificiel (15, 17).

16. Dispositif médical qui est produit par un procédé selon l'une des revendications précédentes, **caractérisé en ce que** les éléments de structure (2) du dispositif médical présentent au moins une paroi latérale inclinée (36) avec une arête de coupe (35) qui est formée entre la paroi latérale (36) et un fond (37) de l'élément de structure (2), dans lequel l'arête de coupe (35) fait saillie au-dessus de la surface de la paroi latérale (36) et présente un flanc coudé qui poursuit de façon continue dans la face de la paroi latérale (36).

17. Composite stratifié (28) muni d'un substrat (3) et d'une couche de matériau sacrificiel (4) pour la production d'un dispositif médical (1) muni d'éléments de structure (2), dans lequel la couche de matériau sacrificiel (4) est disposée sur le substrat (3) et des éléments de masque (10) dans un matériau sacrificiel (9) sont formés sur la couche de matériau sacrificiel (4), dont les flancs présentent un angle (29) qui est complémentaire à l'angle (31) des parois latérales (30) du dispositif (1) à produire, dans lequel l'angle (25) entre les éléments de masque correspond à la largeur (32) d'un élément de structure (2) du dispositif (1) à produire.

18. Composite stratifié (28) muni d'un substrat (3) et d'une couche de matériau sacrificiel (4) pour la production d'un dispositif muni d'éléments de structure (2), en particulier pour la production d'actionneurs et/ou de micro-pompes et/ou d'actionneurs linéaires dans le domaine de la technique de micro-systèmes et/ou d'actionneurs pour des instruments invasifs minimaux et/ou des micro-actionneurs et/ou des micro-antennes flexibles, dans lequel la couche de matériau sacrificiel (4) est disposée sur le substrat (3) et des éléments de masque (10) dans un matériau sacrificiel (9) sont formés sur la couche de matériau sacrificiel (4), dont les flancs présentent un angle (29) qui est complémentaire à l'angle (31) des parois latérales (30) du dispositif à produire, dans lequel l'angle (25) entre les éléments de masque correspond à la largeur (32) d'un élément de structure (2) du dispositif à produire.
